(19) Europäisches Patentamt
European Patent Office
Office européen des brevets

(11) EP 3 848 895 A1

(12) EUROPEAN PATENT APPLICATION
published in accordance with Art. 153(4) EPC

(43) Date of publication:
14.07.2021 Bulletin 2021/28

(51) Int Cl.:
$G06T\ 7/246$ (2017.01)

(21) Application number: 19857194.5

(86) International application number:
PCT/JP2019/034310

(22) Date of filing: 30.08.2019

(87) International publication number:
WO 2020/050187 (12.03.2020 Gazette 2020/11)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 06.09.2018 JP 2018167331

(71) Applicant: Sony Corporation
108-0075 Tokyo (JP)

(72) Inventor: FUKAZAWA, Kentaro
Tokyo 108-0075 (JP)

(74) Representative: D Young & Co LLP
120 Holborn
London EC1N 2DY (GB)

(54) MEDICAL SYSTEM, INFORMATION PROCESSING DEVICE, AND INFORMATION PROCESSING METHOD

(57) A medical system (1) includes irradiation means (11) for irradiating an image capturing target with an electromagnetic wave, image capturing means (12) for capturing a reflected wave caused by the image capturing target irradiated with the electromagnetic wave, acquisition means (1311) for acquiring, from the image capturing means (12), a first image based on a first wavelength band and a second image based on a second wavelength band different from the first wavelength band, first motion estimation means (1312) for calculating a first motion vector as a motion vector between a plurality of the first images on a basis of a feature value in the first image, second motion estimation means (1313) for calculating a second motion vector as a motion vector between a plurality of the second images on a basis of a feature value in the second image, correlation degree calculation means (1314) for calculating a degree of correlation between the first motion vector and the second motion vector, and correction means (1315) for correcting the first image on a basis of the degree of correlation.

FIG.2

**Description**

Field

The present disclosure relates to a medical system, an information processing apparatus, and an information processing method.

Background

In the medical field, a medical doctor and other medical staff visually recognize a lesion or other abnormality in some cases by looking at a special-light image taken by irradiating a living body with special light having a particular wavelength band (e.g., near-infrared light). However, the special-light image taken with special light having a narrower wavelength band than white light is normally darker than a white-light image, so the influence caused by noise is relatively large. Thus, in the special-light image, in one example, weak fluorescence in a deep part of a living body will be buried in noise, causing it to be invisible in some cases.

Thus, the special-light image necessitates noise reduction processing (hereinafter, also referred to as "NR processing"). In addition, in a case where there is a living body movement due to body motion or pulsation in a special-light image, the motion estimation for NR processing is necessary. Accordingly, in one example, an approach of taking a white-light image and a special-light image simultaneously and estimating the motion using the white-light image is developed. This approach is efficient in the case where the motion projected in the special-light image and the motion projected in the white-light image are the same.

Citation List

Patent Literature

Patent Literature 1: JP 5603676 B2

Summary

Technical Problem

However, in performing the NR processing of the special-light image using the approach mentioned above, the difference between the motion projected in the special-light image and the motion projected in the white-light image will cause deterioration in the image quality of the special-light image.

As described above, in the case where two images are taken using two electromagnetic waves with different wavelength bands, the motion vector of one image is used sometimes to perform correction (such as NR processing) the other image. Still, there is room for improvement in terms of accuracy.

Thus, the present disclosure proposes a medical system, an information processing apparatus, and an information processing method, capable of correcting one image with high accuracy on the basis of respective motion vectors of two images taken by using two electromagnetic waves having different wavelength bands.

Solution to Problem

To solve the problem described above, a medical system according to one aspect of the present disclosure includes irradiation means for irradiating an image capturing target with an electromagnetic wave, image capturing means for capturing a reflected wave caused by the image capturing target irradiated with the electromagnetic wave, acquisition means for acquiring, from the image capturing means, a first image based on a first wavelength band and a second image based on a second wavelength band different from the first wavelength band, first motion estimation means for calculating a first motion vector as a motion vector between a plurality of the first images on a basis of a feature value in the first image, second motion estimation means for calculating a second motion vector as a motion vector between a plurality of the second images on a basis of a feature value in the second image, correlation degree calculation means for calculating a degree of correlation between the first motion vector and the second motion vector, and correction means for correcting the first image on a basis of the degree of correlation.

Brief Description of Drawings

FIG. 1 is a diagram illustrating an exemplary configuration of a medical system according to a first embodiment of the present disclosure.

FIG. 2 is a diagram illustrating an exemplary configuration of an information processing apparatus according to the first embodiment of the present disclosure.

FIG. 3 is a schematic diagram illustrating the relationship between signals and magnitudes of noise in special light and white light in the first embodiment of the present disclosure.

FIG. 4 is a schematic view illustrating how an image capturing target is irradiated with special light and white light in the first embodiment of the present disclosure.

FIG. 5 is a diagram illustrated to describe noise reduction processing according to the first embodiment of the present disclosure.

FIG. 6 is a schematic diagram of a white-light image and a special-light image according to the first embodiment of the present disclosure.

FIG. 7 is a diagram illustrated to describe motion correlation between Case 1 and Case 2 in the first embodiment of the present disclosure.

FIG. 8 is a diagram illustrated to describe noise reduction processing according to the first embodiment of the present disclosure.

FIG. 9 is a flowchart illustrating image processing by the information processing apparatus according to the first embodiment of the present disclosure.

FIG. 10 is a graph illustrating the relationship between $\alpha$ and the degree of correlation in a second embodiment of the present disclosure.

FIG. 11 is a flowchart illustrating image processing by the information processing apparatus according to the second embodiment of the present disclosure.

FIG. 12 is a flowchart illustrating image processing by the information processing apparatus according to a third embodiment of the present disclosure.

FIG. 13 is a view illustrating an example of a schematic configuration of an endoscopic surgery system according to application example 1 of the present disclosure.

FIG. 14 is a block diagram illustrating an example of a functional configuration of a camera head and a CCU illustrated in FIG. 13.

FIG. 15 is a view illustrating an example of a schematic configuration of a microscopic surgery system according to application example 2 of the present disclosure.

FIG. 16 is a view illustrating a state of surgery in which the microscopic surgery system illustrated in FIG. 15 is used.

Description of Embodiments

The description is now given of embodiments of the present disclosure in detail with reference to the drawings. Moreover, in embodiments described below, the same components are denoted by the same reference numerals, and so a description thereof is omitted as appropriate.

(First embodiment)

[Configuration of medical system according to first embodiment]

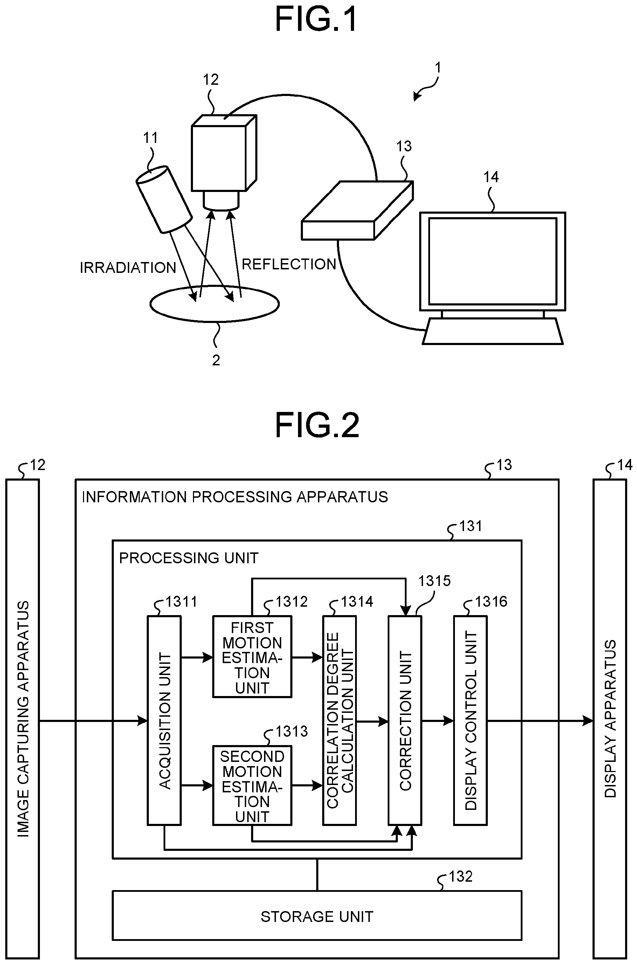

FIG. 1 is a diagram illustrating an exemplary configuration of a medical system 1 according to a first embodiment of the present disclosure. The medical system 1 according to the first embodiment roughly includes at least a light source 11 (irradiation means), an image capturing apparatus 12 (image capturing means), and an information processing apparatus 13. In addition, a display apparatus 14 or the like can be further provided if necessary. Each component is now described in detail.

(1) Light source

The light source 11 includes a first light source that irradiates an image capturing target 2 with special light having a particular wavelength band and a second light source that irradiates the image capturing target 2 with white light. In the first embodiment, the special light is, in one example, near-infrared light. In addition, the image capturing target 2 is irradiated with the special light from the first light source and the white light from the second light source simultaneously.

(2) Image capturing target

The image capturing target 2 can be various, but in one example, a living body is. In one example, the use of the medical system 1 according to the present disclosure in microscopic surgery, endoscopic surgery, or the like makes it possible to perform surgery while checking the blood vessels' position. Thus, it is possible to perform safer and more accurate surgery, leading to a contribution to the further development of medical technology.

(3) Image capturing apparatus

The image capturing apparatus 12 captures the reflected wave from the image capturing target 2 irradiated with electromagnetic waves. The image capturing apparatus 12 includes a special-light image capturing unit that captures a special-light image (first image or near-infrared light image) and a white-light image capturing unit that captures a white-light image (second image). The special-light image capturing unit is, in one example, an infrared (IR) imager. The white-light image capturing unit is, in one example, an RGB (red/green/blue) imager. In this case, the image capturing apparatus 12 includes, in one example, a dichroic mirror as the main configuration in addition to the special-light image capturing unit and the white-light image capturing unit.

As described above, the light source 11 emits special light and white light. The dichroic mirror separates the received light into special light and white light. The special-light image capturing unit captures a special-light image obtained from the special light separated by the dichroic mirror. The white-light image capturing unit captures a white-light image obtained from the white light separated by the dichroic mirror. The image capturing apparatus 12 having such a configuration makes it possible to acquire the special-light image and the white-light image simultaneously. Moreover, the special-light image and the white-light image can be captured by the respective individual image capturing apparatuses.

(4) Information processing apparatus

The description is now given of the information processing apparatus 13 with reference to FIG. 2. FIG. 2 is a diagram illustrating an exemplary configuration of the information processing apparatus 13 according to the first embodiment of the present disclosure. The information processing apparatus 13 is an image processing apparatus and mainly includes a processing unit 131 and a storage unit 132.

The processing unit 131 is configured with, in one example, a central processing unit (CPU). The processing unit 131 includes an acquisition unit 1311 (acquisition means), a first motion estimation unit 1312 (first motion estimation means), a second motion estimation unit 1313 (second motion estimation means), a correlation degree calculation unit 1314 (correlation degree calculation means), a correction unit 1315 (correction means), and a display control unit 1316.

The acquisition unit 1311 acquires a special-light image (the first image based on a first wavelength band) and a white-light image (the second image based on a second wavelength band different from the first wavelength band) from the image capturing apparatus 12.

The first motion estimation unit 1312 calculates a special-light motion vector (first motion vector) that is a motion vector between a plurality of special-light images on the basis of a feature value in the special-light image. The second motion estimation unit 1313 calculates a white-light motion vector (second motion vector) that is a motion vector between a plurality of white-light images on the basis of a feature value in the white-light image.

Examples of a specific technique of performing the motion estimation by the first motion estimation unit 1312 and the second motion estimation unit 1313 can include block (template) matching or gradient-based algorithms but not limited to the example mentioned above. Any technique can be used. In addition, such motion estimation can be performed for each pixel or each block.

The correlation degree calculation unit 1314 calculates the degree of correlation between the special-light motion vector and the white-light motion vector. The correlation degree calculation unit 1314 calculates, in one example, a correlation coefficient between the special-light motion vector and the white-light motion vector as the degree of correlation.

Further, the correlation degree calculation unit 1314 can calculate the sum of absolute values of the differences between the special-light motion vector and the white-light motion vector as the degree of correlation. In addition, the correlation degree calculation unit 1314 can calculate the sum of squares of the differences between the special-light motion vector and the white-light motion vector as the degree of correlation. In addition, a way of calculating the degree of correlation is not limited to the examples mentioned above, and a way of calculating any index capable of evaluating the correlation (similarity) can be used.

The correction unit 1315 corrects the special-light image on the basis of the degree of correlation between the special-light motion vector and the white-light motion vector calculated by the correlation degree calculation unit 1314. In one example, the correction unit 1315 corrects the special-light image on the basis of the white-light motion vector in the case where the degree of correlation is equal to or higher than a predetermined threshold. The correction unit 1315 corrects the special-light image on the basis of the special-light motion vector in the case where the degree of correlation is less than the predetermined threshold. Moreover, the predetermined threshold regarding the degree of correlation is stored in the storage unit 132 in advance.

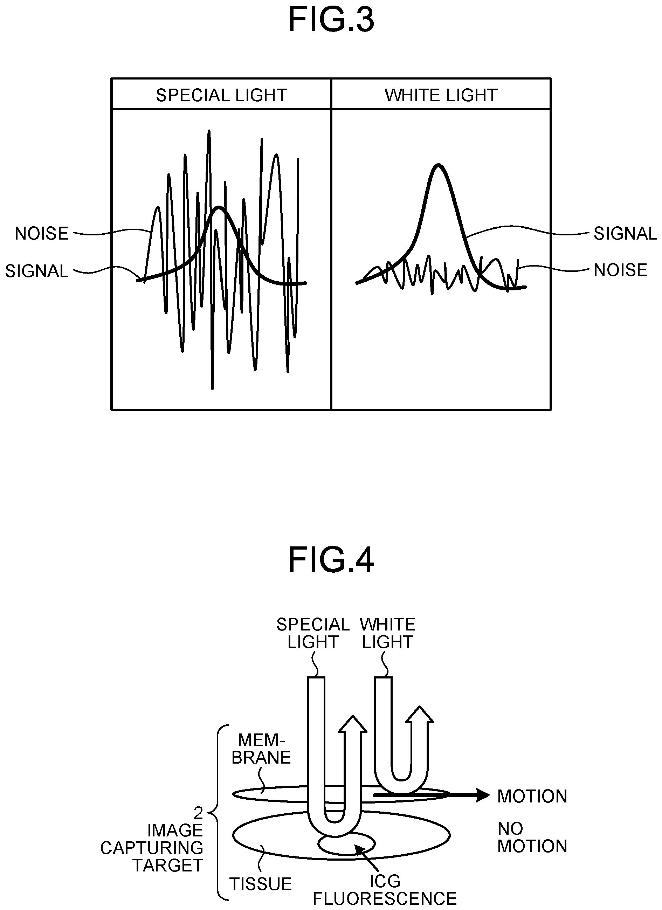

Further, the correction unit 1315 performs, in one example, noise reduction processing of reducing the noise of the special-light image as the processing of correcting the special-light image on the basis of the degree of correlation. The noise reduction processing is now described with reference to FIGS. 3 to 8.

FIG. 3 is a schematic diagram illustrating the relationship between signals and magnitudes of noise in special light and white light in the first embodiment of the present disclosure. As illustrated in FIG. 3, the signal obtained using the special light is relatively smaller in noise than the signal obtained using the white light. Thus, in estimating the motion, it is considered better to use the white-light image than the special-light image. However, this consideration is based on the assumption that the motion projected in the special-light image and the motion projected in the white-light image are the same.

FIG. 4 is a schematic view illustrating how an image capturing target 2 is irradiated with special light and white light in the first embodiment of the present disclosure. It is herein assumed that the tissue of the living body of the image capturing target 2 has a blood vessel that emits light by indocyanine green (ICG) fluorescence. In addition, the tissue is assumed to be covered with a membrane (or such as fat). Then, there are cases where the special light is reflected mainly from the tissue, and the white light is reflected mainly from the membrane. In these cases, in one example, if the membrane is peeled from the tissue, sometimes the membrane moves, but the tissue does not move. In such a case, if motion estimation is performed using the white-light image and the NR processing is performed on the special-light image using an estimation result of the motion, the image quality of the special-light image will deteriorate.

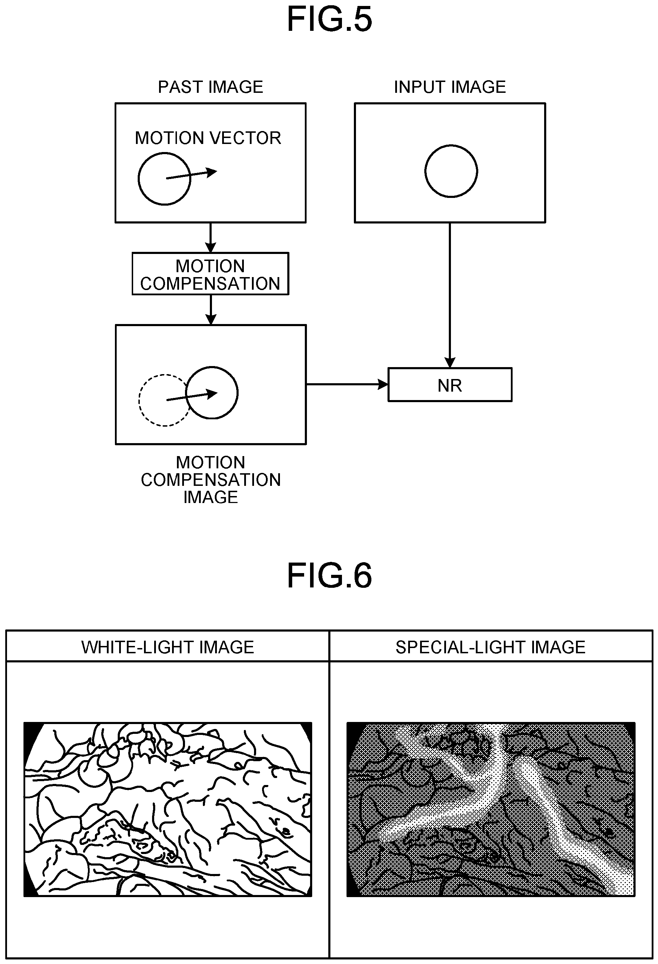

FIG. 5 is a diagram illustrated to describe noise reduction processing according to the first embodiment of the present disclosure. The correction unit 1315 is capable of performing the NR processing using, in one example, an input image (the current image) and a motion compensation image obtained by performing motion compensation on the past image (an image one frame before). The degree of correlation between the special-light motion vector and the white-light motion vector is used to perform the motion compensation with high accuracy (details described later).

FIG. 6 is a schematic diagram of a white-light image and a special-light image according to the first embodiment of the present disclosure. It can be seen that the special-light image is darker overall than the white-light image.

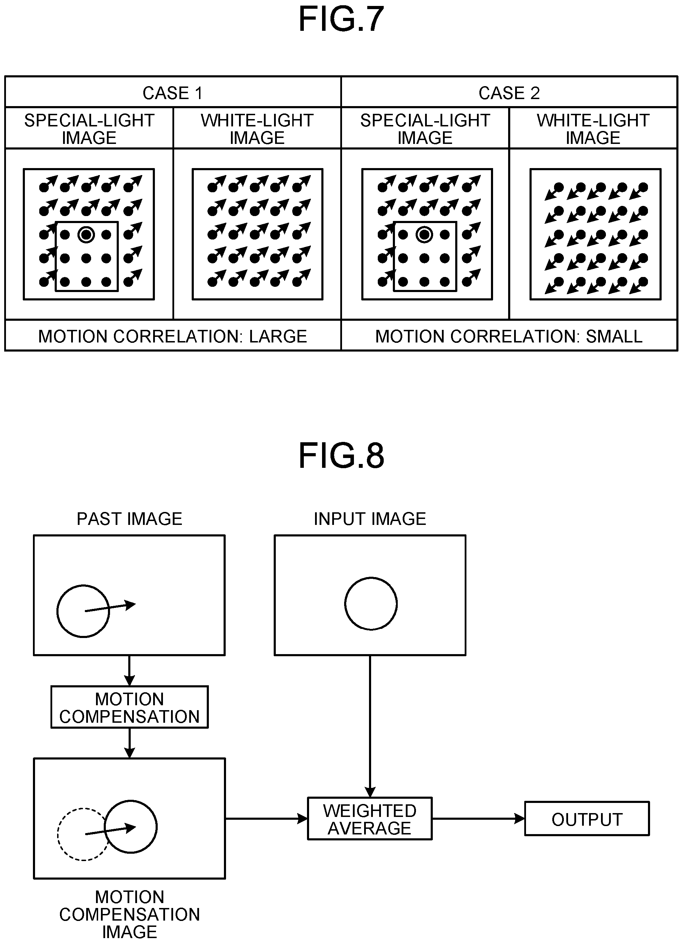

FIG. 7 is a diagram illustrated to describe motion correlation between Case 1 and Case 2 in the first embodiment of the present disclosure. In the special-light image and the white-light image of FIG. 7, black circles are pixels or blocks (collection of a plurality of pixels). The assumption is now given that black circles are pixels.

In Case 1, the event of calculating the degree of correlation for the central pixel of the special-light image is considered. In this case, pixels to which the arrow is added in the special-light image are assumed to be capable of normal motion estimation. In addition, the motion estimation is assumed to be failed due to the reason such as dark 3×3 pixels surrounded by the frame. In addition, in the white-light image, it is possible to estimate the motion for 5×5 pixels. In this case, the correlation degree calculation unit 1314 calculates the degree of correlation between the special-light motion vector and the white-light motion vector by using information regarding the portion whose motion is estimable. In Case 1, the portion whose motion is estimable in the special-light image and the portion whose motion is estimable in the white-light image are the same in motion, so the degree of correlation (motion correlation) is large.

On the other hand, in Case 2, the portion whose motion is estimable in the special-light image and the portion whose motion is estimable in the white-light image are different in motion, so the degree of correlation (motion correlation) is small.

FIG. 8 is a diagram illustrated to describe noise reduction processing according to the first embodiment of the present disclosure. The correction unit 1315 initially performs motion compensation on the past image (an image one frame before). Meanwhile, in this event, in one example, if the degree of correlation is equal to or higher than a predetermined threshold, motion compensation is performed on the basis of the white-light motion vector. In addition, if the degree of correlation is less than the predetermined threshold, the correction unit 1315 performs motion compensation on the basis of the special-light motion vector. This makes it possible to perform motion compensation with high accuracy.

Further, the correction unit 1315 is capable of calculating a weighted average of the motion compensation image and the input image (the current image) to perform the NR processing. The motion compensation is performed with high accuracy, so the NR processing is also performed with high accuracy. Moreover, motion compensation or weight averaging is performed, in one example, in pixel units. In addition, the past image can be an output image one frame before or an input image one frame before.

Referring back to FIG. 2, the display control unit 1316 controls the display apparatus 14 to display the special-light image being corrected (such as being subjected to NR processing) or the like.

The storage unit 132 stores various types of information such as the special-light image and white-light image acquired by the acquisition unit 1311, a calculation result obtained by each unit in the processing unit 131, and a threshold of the degree of correlation. Moreover, an external storage device of the medical system 1 can be used instead of the storage unit 132.

(5) Display apparatus

The control of the display apparatus 14 by the display control unit 1316 allows various types of information such as the special-light image and white-light image acquired by the acquisition unit 1311, a calculation result obtained by each unit in the processing unit 131, and a threshold of the degree of correlation to be displayed. Moreover, an external display apparatus of the medical system 1 can be used instead of the display apparatus 14.

[SC image generation processing according to first embodiment]

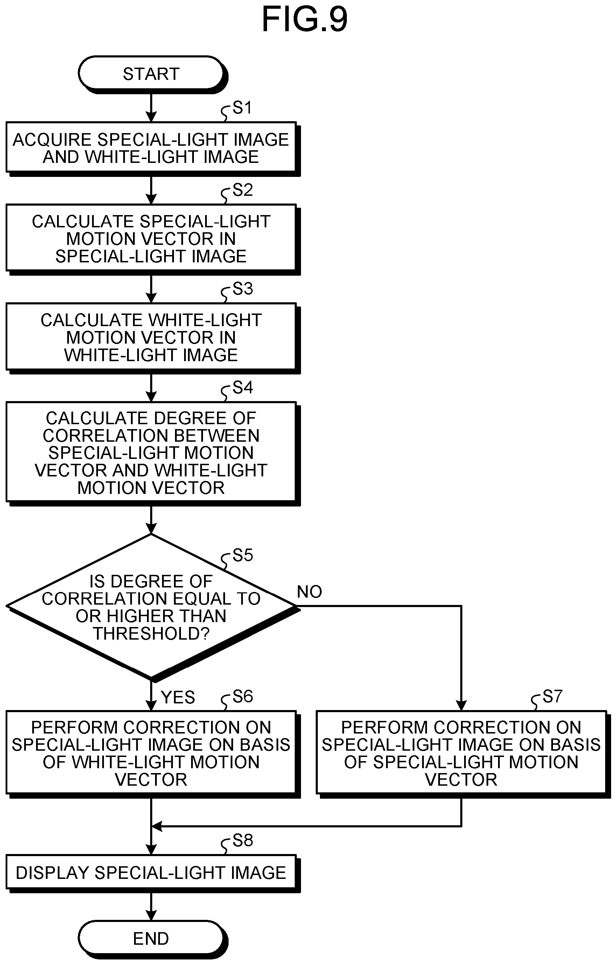

The description is now given of the image processing performed by the information processing apparatus 13 with reference to FIG. 9. FIG. 9 is a flowchart illustrating image processing performed by the information processing apparatus 13 according to the first embodiment of the present disclosure.

In step S1, initially, the acquisition unit 1311 acquires a special-light image and a white-light image from the image capturing apparatus 12.

Subsequently, in step S2, the first motion estimation unit 1312 calculates a special-light motion vector on the basis of a feature value in the special-light image.

Subsequently, in step S3, the second motion estimation unit 1313 calculates a white-light motion vector on the basis of a feature value in the white-light image.

Subsequently, in step S4, the correlation degree calculation unit 1314 calculates the degree of correlation between the special-light motion vector and the white-light motion vector.

Subsequently, in step S5, the correction unit 1315 determines whether or not the degree of correlation is equal to or higher than a predetermined threshold. If the result is Yes, the processing proceeds to step S6, and if the result is No, the processing proceeds to step S7.

In step S6, the correction unit 1315 performs correction (such as NR processing) on the special-light image on the basis of the white-light motion vector.

In step S7, the correction unit 1315 performs correction (such as NR processing) on the special-light image on the basis of the special-light motion vector.

In step S8 following steps S6 and S7, the display control unit 1316 controls the display apparatus 14 to display the special-light image being corrected (such as being subjected to NR processing).

As described above, the information processing apparatus 13 according to the first embodiment makes it possible, on the basis of respective motion vectors of two images taken by using two electromagnetic waves having different wavelength bands, to correct one image with high accuracy. In one example, in using a special-light image and a white-light image, the NR processing on the special-light image is performed using the white-light motion vector if the degree of correlation between the two motion vectors is large. The NR processing on the special-light image is performed using the special-light motion vector if the degree of correlation between the two motion vectors is small. Thus, it is possible to achieve highly accurate NR processing.

In one example, in the neurosurgical and cardiac surgical procedures, fluorescence observation using ICG is generally performed for blood flow observation during surgery. This ICG fluorescence observation is a technique of observing the circulation of blood or lymphatic vessels in a minimally invasive manner by utilizing the characteristics that ICG binds to plasma protein in vivo and emits fluorescence by near-infrared excitation light. In this case, even when there are membranes or fats on the tissue, blood flow, or tumor observed in the special-light image, and the correlation between the motion captured by the special-light image and the motion captured by the white-light image is small such as when membranes or fats are being peeled off, it is possible to perform the NR processing on the special-light image without causing deterioration of image quality. Thus, the possibility that a medical doctor looking at the special-light image subjected to the NR processing makes an erroneous diagnosis is reduced, which leads to safer surgery.

(Second embodiment)

The description is now given of a second embodiment. Descriptions of the same matters as the first embodiment will be omitted as appropriate. In the first embodiment, the correction unit 1315 corrects the special-light image on the basis of the white-light motion vector if the degree of correlation is equal to or higher than a predetermined threshold, and corrects the special-light image on the basis of the special-light motion vector if the degree of correlation is less than the predetermined threshold. In the second embodiment, the correction unit 1315 calculates a third motion vector (MV3) by weighting and summing the special-light motion vector (MV1) and the white-light motion vector (MV2) depending on the degree of correlation and corrects the special-light image on the basis of the third motion vector (MV3), as expressed in Formula (1) as follows:

$$MV3 = (1 - \alpha) \times MV1 + \alpha \times MV2 \ldots \text{Formula (1)}$$

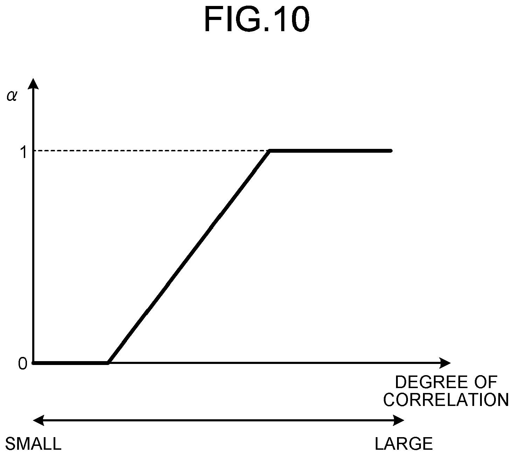

Here, FIG. 10 is a graph illustrating the relationship between $\alpha$ and the degree of correlation in the second embodiment of the present disclosure. The coefficient $\alpha$ (mixing ratio) depending on the degree of correlation is set, as illustrated in FIG. 10. In the graph of FIG. 10, the vertical axis is the value of $\alpha$, and the horizontal axis is the degree of correlation. In this way, in the case of a small degree of correlation, the ratio of the special-light motion vector (MV1) increases. In the case of a large degree of correlation, the ratio of the white-light motion vector (MV2) increases. Accordingly, an appropriate third motion vector (MV3) is obtained.

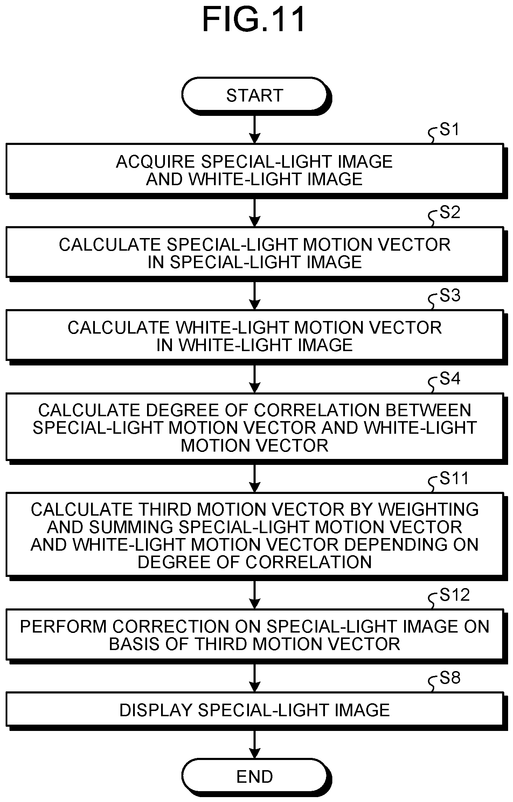

FIG. 11 is a flowchart illustrating the image processing by the information processing apparatus 13 according to the second embodiment of the present disclosure. Steps S1 to S4 are similar to those in FIG. 9.

In step S11 following step S4, the correction unit 1315 calculates the third motion vector (MV3) by weighting and summing the special-light motion vector (MV1) and the white-light motion vector (MV2) depending on the degree of correlation, as expressed Formula (1) above.

Subsequently, in step S12, the correction unit 1315 performs correction (such as NR processing) on the special-light image on the basis of the third motion vector (MV3) .

Subsequently, in step S8, the display control unit 1316 controls the display apparatus 14 to display the special-light image being corrected (such as being subjected to NR processing) or the like.

As described above, according to the information processing apparatus 13 of the second embodiment, in correcting a special-light image, it is possible to use the third motion vector calculated by weighting and summing the special-light motion vector and the white-light motion vector depending on the degree of correlation, rather than the use of only one of the special-light motion vector and the white-light motion vector. Accordingly, it is possible to achieve highly accurate correction.

(Third embodiment)

The description is now given of a third embodiment. Descriptions of the same matters as the first embodiment will be omitted as appropriate. In the third embodiment, the correction unit 1315 performs motion compensation on the special-light image on the basis of the special-light motion vector and performs motion compensation on the white-light image on the basis of the white-light motion vector. The correction unit 1315 generates a third image by weighting and summing the motion-compensated special-light image and the motion-compensated white-light image depending on the degree of correlation. The correction unit 1315 corrects the special-light image on the basis of the third image. In this case, the way of weighting and summing is similar to that of weighting and summing using Formula (1) of the second embodiment. In other words, in the case of a small degree of correlation, the ratio of the motion-compensated special-light image increases, and in the case of a large degree of correlation, the ratio of the motion-compensated white-light image increases. Accordingly, an appropriate third image is obtained.

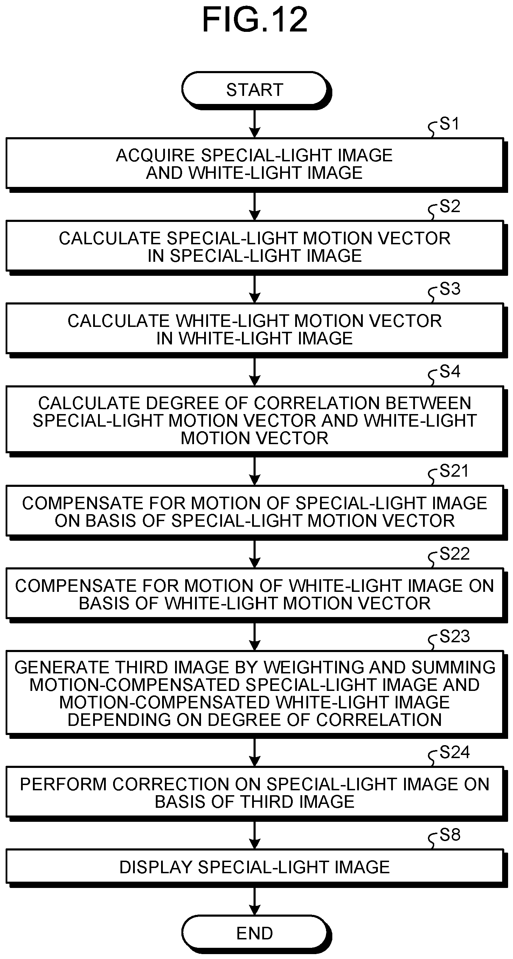

FIG. 12 is a flowchart illustrating the image processing by the information processing apparatus 13 according to the third embodiment of the present disclosure. Steps S1 to S4 are similar to those in FIG. 9.

In step S21 following step S4, the correction unit 1315 compensates for the motion of the special-light image on the basis of the special-light motion vector.

Subsequently, in step S22, the correction unit 1315 compensates for the motion of the white-light image on the basis of the white-light motion vector.

Subsequently, in step S23, the correction unit 1315 generates the third image by weighting and summing the motion-compensated special-light image and the motion-compensated white-light image depending on the degree of correlation.

Subsequently, in step S24, the correction unit 1315 performs correction (such as NR processing) on the special-light image on the basis of the third image.

Subsequently, in step S8, the display control unit 1316 controls the display apparatus 14 to display the special-light image being corrected (such as being subjected to NR processing) or the like.

As described above, according to the information processing apparatus 13 of the third embodiment, in correcting the special-light image, it is possible to perform correction with more accuracy by using the third image calculated by weighting and summing the motion-compensated special-light image and the motion-compensated white-light image.

(Fourth embodiment)

In the first to third embodiments, the first image to be subjected to correction (such as NR processing) is a special-light image, and the other second image is a white-light image. In the fourth embodiment, the first image to be subjected to correction (such as NR processing) is a white-light image, and the other second image is a special-light image.

This makes it possible to perform the correction of the white-light image by using not only the white-light motion vector but also the special-light motion vector. It is useful in the case where the motion vector is recognizable more accurately in the special-light image than in the white-light image depending on the type of the image capturing target 2 or the usage environment of the medical system 1.

(Fifth embodiment)

In the first to fourth embodiments, the first image to be subjected to correction (such as NR processing) and the other second image use a combination of the special-light image and the white-light image. In the fifth embodiment, the first image to be subjected to correction (such as NR processing) and the other second image use two special-light images obtained by being irradiated with two special-light rays having different wavelength bands and taking them.
This makes it possible to use respective motion vectors of any two special-light images to perform correction on one of the special-light images.

(Application example 1)

The technology according to the present disclosure is applicable to various products. In one example, the technology according to the present disclosure is applicable to an endoscopic surgery system.

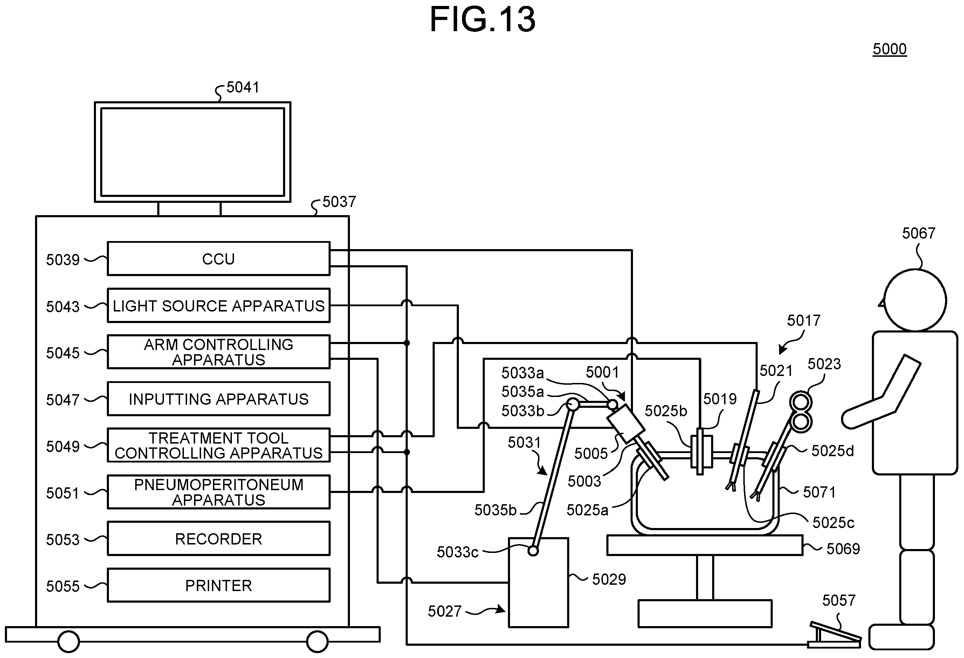

FIG. 13 is a view illustrating an example of a schematic configuration of an endoscopic surgery system 5000 to which the technology according to the present disclosure can be applied. In FIG. 13, a state is illustrated in which a surgeon (medical doctor) 5067 is using the endoscopic surgery system 5000 to perform surgery for a patient 5071 on a patient bed 5069. As illustrated, the endoscopic surgery system 5000 includes an endoscope 5001, other surgical tools 5017, a supporting arm apparatus 5027 which supports the endoscope 5001 thereon, and a cart 5037 on which various apparatus for endoscopic surgery are mounted.
In endoscopic surgery, in place of incision of the abdominal wall to perform laparotomy, a plurality of tubular aperture devices called trocars 5025a to 5025d are used to puncture the abdominal wall. Then, a lens barrel 5003 of the endoscope 5001 and the other surgical tools 5017 are inserted into body cavity of the patient 5071 through the trocars 5025a to 5025d. In the example illustrated, as the other surgical tools 5017, a pneumoperitoneum tube 5019, an energy device 5021 and forceps 5023 are inserted into body cavity of the patient 5071. Further, the energy device 5021 is a treatment tool for performing incision and peeling of a tissue, sealing of a blood vessel or the like by high frequency current or ultrasonic vibration. However, the surgical tools 5017 illustrated are mere examples at all, and as the surgical tools 5017, various surgical tools which are generally used in endoscopic surgery such as, for example, tweezers or a retractor may be used.
An image of a surgical region in a body cavity of the patient 5071 imaged by the endoscope 5001 is displayed on a display apparatus 5041. The surgeon 5067 would use the energy device 5021 or the forceps 5023 while watching the image of the surgical region displayed on the display apparatus 5041 on the real time basis to perform such treatment as, for example, resection of an affected area. It is to be noted that, though not illustrated, the pneumoperitoneum tube 5019, the energy device 5021 and the forceps 5023 are supported by the surgeon 5067, an assistant or the like during surgery.

(Supporting arm apparatus)

The supporting arm apparatus 5027 includes an arm unit 5031 extending from a base unit 5029. In the example illustrated, the arm unit 5031 includes joint portions 5033 a, 5033 b and 5033 c and links 5035a and 5035b and is driven under the control of an arm controlling apparatus 5045. The endoscope 5001 is supported by the arm unit 5031 such that the position and the posture of the endoscope 5001 are controlled. Consequently, stable fixation in position of the endoscope 5001 can be implemented.

(Endoscope)

The endoscope 5001 includes the lens barrel 5003 which has a region of a predetermined length from a distal end thereof to be inserted into a body cavity of the patient 5071, and a camera head 5005 connected to a proximal end of the lens barrel 5003. In the example illustrated, the endoscope 5001 is illustrated as a rigid endoscope having the lens barrel 5003 of the hard type. However, the endoscope 5001 may otherwise be configured as a flexible endoscope having the lens barrel 5003 of the flexible type.
The lens barrel 5003 has, at a distal end thereof, an opening in which an objective lens is fitted. A light source apparatus 5043 is connected to the endoscope 5001 such that light generated by the light source apparatus 5043 is introduced to a distal end of the lens barrel by a light guide extending in the inside of the lens barrel 5003 and is irradiated toward an observation target in a body cavity of the patient 5071 through the objective lens. It is to be noted that the endoscope 5001 may be a forward-viewing endoscope or may be an oblique-viewing endoscope or a side-viewing endoscope.

An optical system and an image pickup element are provided in the inside of the camera head 5005 such that reflected light (observation light) from an observation target is condensed on the image pickup element by the optical system. The observation light is photoelectrically converted by the image pickup element to generate an electric signal corresponding to the observation light, namely, an image signal corresponding to an observation image. The image signal is transmitted as RAW data to a CCU 5039. It is to be noted that the camera head 5005 has a function incorporated therein for suitably driving the optical system of the camera head 5005 to adjust the magnification and the focal distance.

It is to be noted that, in order to establish compatibility with, for example, a stereoscopic vision (three dimensional (3D) display), a plurality of image pickup elements may be provided on the camera head 5005. In this case, a plurality of relay optical systems are provided in the inside of the lens barrel 5003 in order to guide observation light to each of the plurality of image pickup elements.

(Various apparatus incorporated in cart)

The CCU 5039 includes a central processing unit (CPU), a graphics processing unit (GPU) or the like and integrally controls operation of the endoscope 5001 and the display apparatus 5041. In particular, the CCU 5039 performs, for an image signal received from the camera head 5005, various image processes for displaying an image based on the image signal such as, for example, a development process (demosaic process). The CCU 5039 provides the image signal for which the image processes have been performed to the display apparatus 5041. Further, the CCU 5039 transmits a control signal to the camera head 5005 to control driving of the camera head 5005. The control signal may include information relating to an image pickup condition such as a magnification or a focal distance.

The display apparatus 5041 displays an image based on an image signal for which the image processes have been performed by the CCU 5039 under the control of the CCU 5039. If the endoscope 5001 is ready for imaging of a high resolution such as 4K (horizontal pixel number 3840 × vertical pixel number 2160), 8K (horizontal pixel number 7680 × vertical pixel number 4320) or the like and/or ready for 3D display, then a display apparatus by which corresponding display of the high resolution and/or 3D display are possible may be used as the display apparatus 5041. Where the apparatus is ready for imaging of a high resolution such as 4K or 8K, if the display apparatus used as the display apparatus 5041 has a size of equal to or not less than 55 inches, then a more immersive experience can be obtained. Further, a plurality of display apparatus 5041 having different resolutions and/or different sizes may be provided in accordance with purposes.

The light source apparatus 5043 includes a light source such as, for example, a light emitting diode (LED) and supplies irradiation light for imaging of a surgical region to the endoscope 5001.

The arm controlling apparatus 5045 includes a processor such as, for example, a CPU and operates in accordance with a predetermined program to control driving of the arm unit 5031 of the supporting arm apparatus 5027 in accordance with a predetermined controlling method.

An inputting apparatus 5047 is an input interface for the endoscopic surgery system 5000. A user can perform inputting of various kinds of information or instruction inputting to the endoscopic surgery system 5000 through the inputting apparatus 5047. For example, the user would input various kinds of information relating to surgery such as physical information of a patient, information regarding a surgical procedure of the surgery and so forth through the inputting apparatus 5047. Further, the user would input, for example, an instruction to drive the arm unit 5031, an instruction to change an image pickup condition (type of irradiation light, magnification, focal distance or the like) by the endoscope 5001, an instruction to drive the energy device 5021 or the like through the inputting apparatus 5047.

The type of the inputting apparatus 5047 is not limited and may be that of any one of various known inputting apparatus. As the inputting apparatus 5047, for example, a mouse, a keyboard, a touch panel, a switch, a foot switch 5057 and/or a lever or the like may be applied. Where a touch panel is used as the inputting apparatus 5047, it may be provided on the display face of the display apparatus 5041.

Otherwise, the inputting apparatus 5047 is a device to be mounted on a user such as, for example, a glasses type wearable device or a head mounted display (HMD), and various kinds of inputting are performed in response to a gesture or a line of sight of the user detected by any of the devices mentioned. Further, the inputting apparatus 5047 includes a camera which can detect a motion of a user, and various kinds of inputting are performed in response to a gesture or a line of sight of a user detected from a video imaged by the camera. Further, the inputting apparatus 5047 includes a microphone which can collect the voice of a user, and various kinds of inputting are performed by voice collected by the microphone. By configuring the inputting apparatus 5047 such that various kinds of information can be inputted in a contactless fashion in this manner, especially a user who belongs to a clean area (for example, the surgeon 5067) can operate an apparatus belonging to an unclean area in a contactless fashion. Further, since the user can operate an apparatus without releasing a possessed surgical tool from its hand, the convenience to the user is improved.

A treatment tool controlling apparatus 5049 controls driving of the energy device 5021 for cautery or incision of a tissue, sealing of a blood vessel or the like. A pneumoperitoneum apparatus 5051 feeds gas into a body cavity of the patient 5071 through the pneumoperitoneum tube 5019 to inflate the body cavity in order to secure the field of view of the endoscope 5001 and secure the working space for the surgeon. A recorder 5053 is an apparatus capable of recording various kinds of information relating to surgery. A printer 5055 is an apparatus capable of printing various kinds of information relating to surgery in various forms such as a text, an image or a graph.

In the following, especially a characteristic configuration of the endoscopic surgery system 5000 is described in more detail.

(Supporting arm apparatus)

The supporting arm apparatus 5027 includes the base unit 5029 serving as a base, and the arm unit 5031 extending from the base unit 5029. In the example illustrated, the arm unit 5031 includes the plurality of joint portions 5033a, 5033b and 5033c and the plurality of links 5035a and 5035b connected to each other by the joint portion 5033b. In FIG. 13, for simplified illustration, the configuration of the arm unit 5031 is illustrated in a simplified form. Actually, the shape, number and arrangement of the joint portions 5033a to 5033c and the links 5035a and 5035b and the direction and so forth of axes of rotation of the joint portions 5033a to 5033c can be set suitably such that the arm unit 5031 has a desired degree of freedom. For example, the arm unit 5031 may preferably be configured such that it has a degree of freedom equal to or not less than 6 degrees of freedom. This makes it possible to move the endoscope 5001 freely within the movable range of the arm unit 5031. Consequently, it becomes possible to insert the lens barrel 5003 of the endoscope 5001 from a desired direction into a body cavity of the patient 5071.

An actuator is provided in each of the joint portions 5033a to 5033c, and the joint portions 5033a to 5033c are configured such that they are rotatable around predetermined axes of rotation thereof by driving of the respective actuators. The driving of the actuators is controlled by the arm controlling apparatus 5045 to control the rotational angle of each of the joint portions 5033 a to 5033c thereby to control driving of the arm unit 5031. Consequently, control of the position and the posture of the endoscope 5001 can be implemented. Thereupon, the arm controlling apparatus 5045 can control driving of the arm unit 5031 by various known controlling methods such as force control or position control.

For example, if the surgeon 5067 suitably performs operation inputting through the inputting apparatus 5047 (including the foot switch 5057), then driving of the arm unit 5031 may be controlled suitably by the arm controlling apparatus 5045 in response to the operation input to control the position and the posture of the endoscope 5001. After the endoscope 5001 at the distal end of the arm unit 5031 is moved from an arbitrary position to a different arbitrary position by the control just described, the endoscope 5001 can be supported fixedly at the position after the movement. It is to be noted that the arm unit 5031 may be operated in a master-slave fashion. In this case, the arm unit 5031 may be remotely controlled by the user through the inputting apparatus 5047 which is placed at a place remote from the operating room.

Further, where force control is applied, the arm controlling apparatus 5045 may perform power-assisted control to drive the actuators of the joint portions 5033a to 5033c such that the arm unit 5031 may receive external force by the user and move smoothly following the external force. This makes it possible to move, when the user directly touches with the arm unit 5031 and moves the arm unit 5031, the arm unit 5031 with comparatively weak force. Accordingly, it becomes possible for the user to move the endoscope 5001 more intuitively by a simpler and easier operation, and the convenience to the user can be improved.

Here, generally in endoscopic surgery, the endoscope 5001 is supported by a medical doctor called scopist. In contrast, where the supporting arm apparatus 5027 is used, the position of the endoscope 5001 can be fixed more certainly without hands, and therefore, an image of a surgical region can be obtained stably and surgery can be performed smoothly.

It is to be noted that the arm controlling apparatus 5045 may not necessarily be provided on the cart 5037. Further, the arm controlling apparatus 5045 may not necessarily be a single apparatus. For example, the arm controlling apparatus 5045 may be provided in each of the joint portions 5033 a to 5033 c of the arm unit 5031 of the supporting arm apparatus 5027 such that the plurality of arm controlling apparatus 5045 cooperate with each other to implement driving control of the and unit 5031.

(Light source apparatus)

The light source apparatus 5043 supplies irradiation light upon imaging of a surgical region to the endoscope 5001. The light source apparatus 5043 includes a white light source which includes, for example, an LED, a laser light source or a combination of them. In this case, where a white light source includes a combination of red, green, and blue (RGB) laser light sources, since the output intensity and the output timing can be controlled with a high degree of accuracy for each color (each wavelength), adjustment of the white balance of a picked up image can be performed by the light source apparatus 5043. Further, in this case, if laser beams from the respective RGB laser light sources are irradiated time-divisionally on an observation target and driving of the image pickup elements of the camera head 5005 is controlled in synchronism with the irradiation timings, then images individually corresponding to the R, G and B colors can be picked up time-divisionally. According to the method just described, a color image can be obtained even if a color filter is not provided for the image pickup element.

Further, driving of the light source apparatus 5043 may be controlled such that the intensity of light to be outputted is changed for each predetermined time. By controlling driving of the image pickup element of the camera head 5005 in synchronism with the timing of the change of the intensity of light to acquire images time-divisionally and synthesizing the images, an image of a high dynamic range free from underexposed blocked up shadows and overexposed highlights can be created.

Further, the light source apparatus 5043 may be configured to supply light of a predetermined wavelength band ready for special light observation. In special light observation, for example, by utilizing the wavelength dependency of absorption of light in a body tissue to irradiate light of a narrower wavelength band in comparison with irradiation light upon ordinary observation (namely, white light), narrow band light observation (narrow band imaging) of imaging a predetermined tissue such as a blood vessel of a superficial portion of the mucous membrane or the like in a high contrast is performed. Alternatively, in special light observation, fluorescent observation for obtaining an image from fluorescent light generated by irradiation of excitation light may be performed. In fluorescent observation, it is possible to perform observation of fluorescent light from a body tissue by irradiating excitation light on the body tissue (autofluorescence observation) or to obtain a fluorescent light image by locally injecting a reagent such as indocyanine green (ICG) into a body tissue and irradiating excitation light corresponding to a fluorescent light wavelength of the reagent upon the body tissue. The light source apparatus 5043 can be configured to supply such narrow-band light and/or excitation light suitable for special light observation as described above.

(Camera head and CCU)

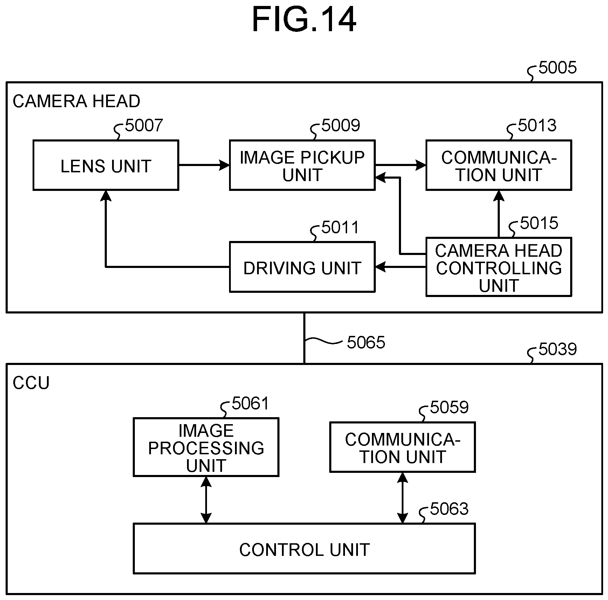

Functions of the camera head 5005 of the endoscope 5001 and the CCU 5039 are described in more detail with reference to FIG. 14. FIG. 14 is a block diagram illustrating an example of a functional configuration of the camera head 5005 and the CCU 5039 illustrated in FIG. 13.

Referring to FIG. 14, the camera head 5005 has, as functions thereof, a lens unit 5007, an image pickup unit 5009, a driving unit 5011, a communication unit 5013 and a camera head controlling unit 5015. Further, the CCU 5039 has, as functions thereof, a communication unit 5059, an image processing unit 5061 and a control unit 5063. The camera head 5005 and the CCU 5039 are connected to be bidirectionally communicable to each other by a transmission cable 5065.

First, a functional configuration of the camera head 5005 is described. The lens unit 5007 is an optical system provided at a connecting location of the camera head 5005 to the lens barrel 5003. Observation light taken in from a distal end of the lens barrel 5003 is introduced into the camera head 5005 and enters the lens unit 5007. The lens unit 5007 includes a combination of a plurality of lenses including a zoom lens and a focusing lens. The lens unit 5007 has optical properties adjusted such that the observation light is condensed on a light receiving face of the image pickup element of the image pickup unit 5009. Further, the zoom lens and the focusing lens are configured such that the positions thereof on their optical axis are movable for adjustment of the magnification and the focal point of a picked up image.

The image pickup unit 5009 includes an image pickup element and disposed at a succeeding stage to the lens unit 5007. Observation light having passed through the lens unit 5007 is condensed on the light receiving face of the image pickup element, and an image signal corresponding to the observation image is generated by photoelectric conversion of the image pickup element. The image signal generated by the image pickup unit 5009 is provided to the communication unit 5013.

As the image pickup element which is included by the image pickup unit 5009, an image sensor, for example, of the complementary metal oxide semiconductor (CMOS) type is used which has a Bayer array and is capable of picking up an image in color. It is to be noted that, as the image pickup element, an image pickup element may be used which is ready, for example, for imaging of an image of a high resolution equal to or not less than 4K. If an image of a surgical region is obtained in a high resolution, then the surgeon 5067 can comprehend a state of the surgical region in enhanced details and can proceed with the surgery more smoothly.

Further, the image pickup element which is included by the image pickup unit 5009 includes such that it has a pair of image pickup elements for acquiring image signals for the right eye and the left eye compatible with 3D display. Where 3D display is applied, the surgeon 5067 can comprehend the depth of a living body tissue in the surgical region more accurately. It is to be noted that, if the image pickup unit 5009 is configured as that of the multi-plate type, then a plurality of systems of lens units 5007 are provided corresponding to the individual image pickup elements of the image pickup unit 5009.

The image pickup unit 5009 may not necessarily be provided on the camera head 5005. For example, the image pickup unit 5009 may be provided just behind the objective lens in the inside of the lens barrel 5003.

The driving unit 5011 includes an actuator and moves the zoom lens and the focusing lens of the lens unit 5007 by a predetermined distance along the optical axis under the control of the camera head controlling unit 5015. Consequently, the magnification and the focal point of a picked up image by the image pickup unit 5009 can be adjusted suitably.

The communication unit 5013 includes a communication apparatus for transmitting and receiving various kinds of information to and from the CCU 5039. The communication unit 5013 transmits an image signal acquired from the image pickup unit 5009 as RAW data to the CCU 5039 through the transmission cable 5065. Thereupon, in order to display a picked up image of a surgical region in low latency, preferably the image signal is transmitted by optical communication. This is because, upon surgery, the surgeon 5067 performs surgery while observing the state of an affected area through a picked up image, it is demanded for a moving image of the surgical region to be displayed on the real time basis as far as possible in order to achieve surgery with a higher degree of safety and certainty. Where optical communication is applied, a photoelectric conversion module for converting an electric signal into an optical signal is provided in the communication unit 5013. After the image signal is converted into an optical signal by the photoelectric conversion module, it is transmitted to the CCU 5039 through the transmission cable 5065.

Further, the communication unit 5013 receives a control signal for controlling driving of the camera head 5005 from the CCU 5039. The control signal includes information relating to image pickup conditions such as, for example, information that a frame rate of a picked up image is designated, information that an exposure value upon image picking up is designated and/or information that a magnification and a focal point of a picked up image are designated. The communication unit 5013 provides the received control signal to the camera head controlling unit 5015. It is to be noted that also the control signal from the CCU 5039 may be transmitted by optical communication. In this case, a photoelectric conversion module for converting an optical signal into an electric signal is provided in the communication unit 5013. After the control signal is converted into an electric signal by the photoelectric conversion module, it is provided to the camera head controlling unit 5015.

It is to be noted that the image pickup conditions such as the frame rate, exposure value, magnification or focal point are set automatically by the control unit 5063 of the CCU 5039 on the basis of an acquired image signal. In other words, an auto exposure (AE) function, an auto focus (AF) function and an auto white balance (AWB) function are incorporated in the endoscope 5001.

The camera head controlling unit 5015 controls driving of the camera head 5005 on the basis of a control signal from the CCU 5039 received through the communication unit 5013. For example, the camera head controlling unit 5015 controls driving of the image pickup element of the image pickup unit 5009 on the basis of information that a frame rate of a picked up image is designated and/or information that an exposure value upon image picking up is designated. Further, for example, the camera head controlling unit 5015 controls the driving unit 5011 to suitably move the zoom lens and the focus lens of the lens unit 5007 on the basis of information that a magnification and a focal point of a picked up image are designated. The camera head controlling unit 5015 may further include a function for storing information for identifying the lens barrel 5003 and/or the camera head 5005.

It is to be noted that, by disposing the components such as the lens unit 5007 and the image pickup unit 5009 in a sealed structure having high airtightness and waterproof, the camera head 5005 can be provided with resistance to an autoclave sterilization process.

Now, a functional configuration of the CCU 5039 is described. The communication unit 5059 includes a communication apparatus for transmitting and receiving various kinds of information to and from the camera head 5005. The communication unit 5059 receives an image signal transmitted thereto from the camera head 5005 through the transmission cable 5065. Thereupon, the image signal may be transmitted preferably by optical communication as described above. In this case, for the compatibility with optical communication, the communication unit 5059 includes a photoelectric conversion module for converting an optical signal into an electric signal. The communication unit 5059 provides the image signal after conversion into an electric signal to the image processing unit 5061.

Further, the communication unit 5059 transmits, to the camera head 5005, a control signal for controlling driving of the camera head 5005. The control signal may also be transmitted by optical communication.

The image processing unit 5061 performs various image processes for an image signal in the form of RAW data transmitted thereto from the camera head 5005. The image processes include various known signal processes such as, for example, a development process, an image quality improving process (a band width enhancement process, a super-resolution process, a noise reduction (NR) process and/or an image stabilization process) and/or an enlargement process (electronic zooming process). Further, the image processing unit 5061 performs a detection process for an image signal in order to perform AE, AF and AWB.

The image processing unit 5061 includes a processor such as a CPU or a GPU, and when the processor operates in accordance with a predetermined program, the image processes and the detection process described above can be performed. It is to be noted that, where the image processing unit 5061 includes a plurality of GPUs, the image processing unit 5061 suitably divides information relating to an image signal such that image processes are performed in parallel by the plurality of GPUs.

The control unit 5063 performs various kinds of control relating to image picking up of a surgical region by the endoscope 5001 and display of the picked up image. For example, the control unit 5063 generates a control signal for controlling driving of the camera head 5005. Thereupon, if image pickup conditions are inputted by the user, then the control unit 5063 generates a control signal on the basis of the input by the user. Alternatively, where the endoscope 5001 has an AE function, an AF function and an AWB function incorporated therein, the control unit 5063 suitably calculates an optimum exposure value, focal distance and white balance in response to a result of a detection process by the image processing unit 5061 and generates a control signal.

Further, the control unit 5063 controls the display apparatus 5041 to display an image of a surgical region on the basis of an image signal for which image processes have been performed by the image processing unit 5061. Thereupon, the control unit 5063 recognizes various objects in the surgical region image using various image recognition technologies. For example, the control unit 5063 can recognize a surgical tool such as forceps, a particular living body region, bleeding, mist when the energy device 5021 is used and so forth by detecting the shape, color and so forth of edges of the objects included in the surgical region image. The control unit 5063 causes, when it controls the display apparatus 5041 to display a surgical region image, various kinds of surgery supporting information to be displayed in an overlapping manner with an image of the surgical region using a result of the recognition. Where surgery supporting information is displayed in an overlapping manner and presented to the surgeon 5067, the surgeon 5067 can proceed with the surgery more safety and certainty.

The transmission cable 5065 which connects the camera head 5005 and the CCU 5039 to each other is an electric signal cable ready for communication of an electric signal, an optical fiber ready for optical communication or a composite cable ready for both of electrical and optical communication.

Here, while, in the example illustrated, communication is performed by wired communication using the transmission cable 5065, the communication between the camera head 5005 and the CCU 5039 may be performed otherwise by wireless communication. Where the communication between the camera head 5005 and the CCU 5039 is performed by wireless communication, there is no necessity to lay the transmission cable 5065 in the operating room. Therefore, such a situation that movement of medical staff in the operating room is disturbed by the transmission cable 5065 can be eliminated.

An example of the endoscopic surgery system 5000 to which the technology according to an embodiment of the present disclosure can be applied has been described above. It is to be noted here that, although the endoscopic surgery system 5000 has been described as an example, the system to which the technology according to an embodiment of the present disclosure can be applied is not limited to the example. For example, the technology according to an embodiment of the present disclosure may be applied to a flexible endoscopic surgery system for inspection or a microscopic surgery system that will be described in application example 2 below,

The technology according to the present disclosure is suitably applicable to the endoscope 5001 among the configurations described above. Specifically, the technology according to the present disclosure is applicable in the case where the image of the surgical site in the body cavity of the patient 5071 taken by the endoscope 5001 is displayed on the display apparatus 5041. The technology according to the present disclosure applied to the endoscope 5001 makes it possible, even if the motion projected in the special-light image and the motion projected in the white-light image are different, to use the respective motion vectors appropriately depending on the degree of correlation of the respective motion vectors of both images. Thus, it is possible to perform correction (such as NR processing) on the special-light image with high accuracy. This allows the surgeon 5067 to observe the surgical site image being corrected (such as being subjected to NR processed) with high accuracy in real-time on the display apparatus 5041, leading to safer surgery.


(Application example 2)

Further, the technology according to the present disclosure may be applied to a microscopic surgery system used for so-called microsurgery that is performed while enlarging a minute region of a patient for observation.

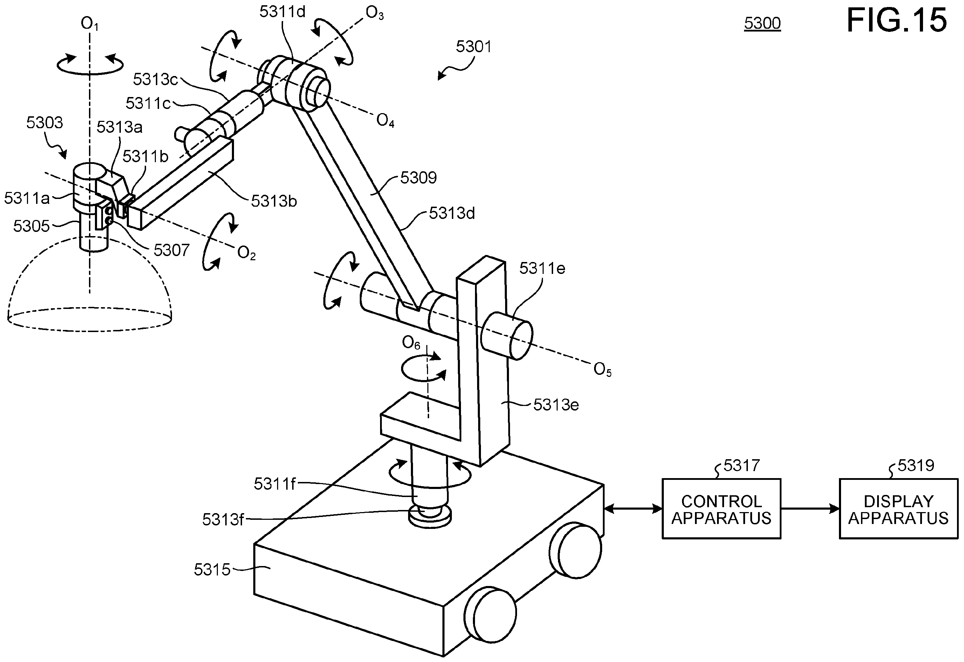

FIG. 15 is a view illustrating an example of a schematic configuration of a microscopic surgery system 5300 to which the technology according to the present disclosure can be applied. Referring to FIG. 15, the microscopic surgery system 5300 includes a microscope apparatus 5301, a control apparatus 5317 and a display apparatus 5319. It is to be noted that, in the description of the microscopic surgery system 5300, the term "user" signifies an arbitrary one of medical staff members such as a surgery or an assistant who uses the microscopic surgery system 5300.

The Microscope apparatus 5301 has a microscope unit 5303 for enlarging an observation target (surgical region of a patient) for observation, an arm unit 5309 which supports the microscope unit 5303 at a distal end thereof, and a base unit 5315 which supports a proximal end of the arm unit 5309.

The microscope unit 5303 includes a cylindrical portion 5305 of a substantially cylindrical shape, an image pickup unit (not illustrated) provided in the inside of the cylindrical portion 5305, and an operation unit 5307 provided in a partial region of an outer circumference of the cylindrical portion 5305. The microscope unit 5303 is a microscope unit of the electronic image pickup type (microscope unit of the video type) which picks up an image electronically by the image pickup unit.

A cover glass member for protecting the internal image pickup unit is provided at an opening face of a lower end of the cylindrical portion 5305. Light from an observation target (hereinafter referred to also as observation light) passes through the cover glass member and enters the image pickup unit in the inside of the cylindrical portion 5305. It is to be noted that a light source includes, for example, a light emitting diode (LED) or the like may be provided in the inside of the cylindrical portion 5305, and upon image picking up, light may be irradiated upon an observation target from the light source through the cover glass member.

The image pickup unit includes an optical system which condenses observation light, and an image pickup element which receives the observation light condensed by the optical system. The optical system includes a combination of a plurality of lenses including a zoom lens and a focusing lens. The optical system has optical properties adjusted such that the observation light is condensed to be formed image on a light receiving face of the image pickup element. The image pickup element receives and photoelectrically converts the observation light to generate a signal corresponding to the observation light, namely, an image signal corresponding to an observation image. As the image pickup element, for example, an image pickup element which has a Bayer array and is capable of picking up an image in color is used. The image pickup element may be any of various known image pickup elements such as a complementary metal oxide semiconductor (CMOS) image sensor or a charge coupled device (CCD) image sensor. The image signal generated by the image pickup element is transmitted as RAW data to the control apparatus 5317. Here, the transmission of the image signal may be performed suitably by optical communication. This is because, since, at a surgery site, the surgeon performs surgery while observing the state of an affected area through a picked up image, in order to achieve surgery with a higher degree of safety and certainty, it is demanded for a moving image of the surgical region to be displayed on the real time basis as far as possible. Where optical communication is used to transmit the image signal, the picked up image can be displayed with low latency.

It is to be noted that the image pickup unit may have a driving mechanism for moving the zoom lens and the focusing lens of the optical system thereof along the optical axis. Where the zoom lens and the focusing lens are moved suitably by the driving mechanism, the magnification of the picked up image and the focal distance upon image picking up can be adjusted. Further, the image pickup unit may incorporate therein various functions which may be provided generally in a microscopic unit of the electronic image pickup such as an auto exposure (AE) function or an auto focus (AF) function.

Further the image pickup unit may be configured as an image pickup unit of the single-plate type which includes a single image pickup element or may be configured as an image pickup unit of the multi-plate type which includes a plurality of image pickup elements. Where the image pickup unit is configured as that of the multi-plate type, for example, image signals corresponding to red, green, and blue colors may be generated by the image pickup elements and may be synthesized to obtain a color image. Alternatively, the image pickup unit may be configured such that it has a pair of image pickup elements for acquiring image signals for the right eye and the left eye compatible with a stereoscopic vision (three dimensional (3D) display). Where 3D display is applied, the surgeon can comprehend the depth of a living body tissue in the surgical region with a higher degree of accuracy. It is to be noted that, if the image pickup unit is configured as that of stereoscopic type, then a plurality of optical systems are provided corresponding to the individual image pickup elements.

The operation unit 5307 includes, for example, a cross lever, a switch or the like and accepts an operation input of the user. For example, the user can input an instruction to change the magnification of the observation image and the focal distance to the observation target through the operation unit 5307. The magnification and the focal distance can be adjusted by the driving mechanism of the image pickup unit suitably moving the zoom lens and the focusing lens in accordance with the instruction. Further, for example, the user can input an instruction to switch the operation mode of the arm unit 5309 (an all-free mode and a fixed mode hereinafter described) through the operation unit 5307. It is to be noted that when the user intends to move the microscope unit 5303, it is supposed that the user moves the microscope unit 5303 in a state in which the user grasps the microscope unit 5303 holding the cylindrical portion 5305. Accordingly, the operation unit 5307 is preferably provided at a position at which it can be operated readily by the fingers of the user with the cylindrical portion 5305 held such that the operation unit 5307 can be operated even while the user is moving the cylindrical portion 5305.

The arm unit 5309 is configured such that a plurality of links (first link 5313a to sixth link 5313f) are connected for rotation relative to each other by a plurality of joint portions (first joint portion 5311a to sixth joint portion 5311f),

The first joint portion 5311a has a substantially columnar shape and supports, at a distal end (lower end) thereof, an upper end of the cylindrical portion 5305 of the microscope unit 5303 for rotation around an axis of rotation (first axis O1) parallel to the center axis of the cylindrical portion 5305. Here, the first joint portion 5311a may be configured such that the first axis O1 thereof is in alignment with the optical axis of the image pickup unit of the microscope unit 5303. By the configuration, if the microscope unit 5303 is rotated around the first axis O1, then the field of view can be changed so as to rotate the picked up image.

The first link 5313a fixedly supports, at a distal end thereof, the first joint portion 5311a. Specifically, the first link 5313a is a bar-like member having a substantially L shape and is connected to the first joint portion 5311a such that one side at the distal end side thereof extends in a direction orthogonal to the first axis O1 and an end portion of the one side abuts with an upper end portion of an outer periphery of the first joint portion 5311a. The second joint portion 5311b is connected to an end portion of the other side on the proximal end side of the substantially L shape of the first link 5313a.

The second joint portion 5311b has a substantially columnar shape and supports, at a distal end thereof, a proximal end of the first link 5313a for rotation around an axis of rotation (second axis O2) orthogonal to the first axis O1. The second link 5313h is fixedly connected at a distal end thereof to a proximal end of the second joint portion 5311b.

The second link 5313b is a bar-like member having a substantially L shape, and one side of a distal end side of the second link 5313b extends in a direction orthogonal to the second axis O2 and an end portion of the one side is fixedly connected to a proximal end of the second joint portion 5311b. The third joint portion 5311c is connected to the other side at the proximal end side of the substantially L shape of the second link 5313b.

The third joint portion 5311c has a substantially columnar shape and supports, at a distal end thereof, a proximal end of the second link 5313b for rotation around an axis of rotation (third axis O3) orthogonal to the first axis O1 and the second axis O2. The third link 5313c is fixedly connected at a distal end thereof to a proximal end of the third joint portion 5311c. By rotating the components at the distal end side including the microscope unit 5303 around the second axis O2 and the third axis O3, the microscope unit 5303 can be moved such that the position of the microscope unit 5303 is changed within a horizontal plane. In other words, by controlling the rotation around the second axis O2 and the third axis O3, the field of view of the picked up image can be moved within a plane.

The third link 5313c is configured such that the distal end side thereof has a substantially columnar shape, and a proximal end of the third joint portion 5311c is fixedly connected to the distal end of the columnar shape such that both of them have a substantially same center axis. The proximal end side of the third link 5313c has a prismatic shape, and the fourth joint portion 5311d is connected to an end portion of the third link 5313c.

The fourth joint portion 5311d has a substantially columnar shape and supports, at a distal end thereof, a proximal end of the third link 5313c for rotation around an axis of rotation (fourth axis O4) orthogonal to the third axis O3. The fourth link 5313d is fixedly connected at a distal end thereof to a proximal end of the fourth joint portion 5311d.

The fourth link 5313d is a bar-like member extending substantially linearly and is fixedly connected to the fourth joint portion 5311d such that it extends orthogonally to the fourth axis O4 and abuts at an end portion of the distal end thereof with a side face of the substantially columnar shape of the fourth joint portion 5311d. The fifth joint portion 5311e is connected to a proximal end of the fourth link 5313d.

The fifth joint portion 5311e has a substantially columnar shape and supports, at a distal end side thereof, a proximal end of the fourth link 5313d for rotation around an axis of rotation (fifth axis O5) parallel to the fourth axis O4. The fifth link 5313e is fixedly connected at a distal end thereof to a proximal end of the fifth joint portion 5311e. The fourth axis O4 and the fifth axis O5 are axes of rotation around which the microscope unit 5303 can be moved in the upward and downward direction. By rotating the components at the distal end side including the microscope unit 5303 around the fourth axis O4 and the fifth axis O5, the height of the microscope unit 5303, namely, the distance between the microscope unit 5303 and an observation target, can be adjusted.

The fifth link 5313e includes a combination of a first member having a substantially L shape one side of which extends in the vertical direction and the other side of which extends in the horizontal direction, and a bar-like second member extending vertically downwardly from the portion of the first member which extends in the horizontal direction. The fifth joint portion 5311e is fixedly connected at a proximal end thereof to a neighboring upper end of a part extending the first member of the fifth link 5313e in the vertical direction. The sixth joint portion 5311f is connected to proximal end (lower end) of the second member of the fifth link 5313e.

The sixth joint portion 5311f has a substantially columnar shape and supports, at a distal end side thereof, a proximal end of the fifth link 5313e for rotation around an axis of rotation (sixth axis O6) parallel to the vertical direction. The sixth link 5313f is fixedly connected at a distal end thereof to a proximal end of the sixth joint portion 5311f.

The sixth link 5313f is a bar-like member extending in the vertical direction and is fixedly connected at a proximal end thereof to an upper face of the base unit 5315.

The first joint portion 5311a to sixth joint portion 5311f have movable ranges suitably set such that the microscope unit 5303 can make a desired movement. Consequently, in the arm unit 5309 having the configuration described above, a movement of totaling six degrees of freedom including three degrees of freedom for translation and three degrees of freedom for rotation can be implemented with regard to a movement of the microscope unit 5303. By configuring the arm unit 5309 such that six degrees of freedom are implemented for movements of the microscope unit 5303 in this manner, the position and the posture of the microscope unit 5303 can be controlled freely within the movable range of the arm unit 5309. Accordingly, it is possible to observe a surgical region from every angle, and surgery can be executed more smoothly.

It is to be noted that the configuration of the arm unit 5309 as illustrated is an example at all, and the number and shape (length) of the links including the arm unit 5309 and the number, location, direction of the axis of rotation and so forth of the joint portions may be designed suitably such that desired degrees of freedom can be implemented. For example, in order to freely move the microscope unit 5303, preferably the arm unit 5309 is configured so as to have six degrees of freedom as described above. However, the arm unit 5309 may also be configured so as to have much greater degree of freedom (namely, redundant degree of freedom). Where a redundant degree of freedom exists, in the arm unit 5309, it is possible to change the posture of the arm unit 5309 in a state in which the position and the posture of the microscope unit 5303 are fixed. Accordingly, control can be implemented which is higher in convenience to the surgeon such as to control the posture of the arm unit 5309 such that, for example, the arm unit 5309 does not interfere with the field of view of the surgeon who watches the display apparatus 5319.

Here, an actuator in which a driving mechanism such as a motor, an encoder which detects an angle of rotation at each joint portion and so forth are incorporated may be provided for each of the first joint portion 5311a to sixth joint portion 5311f. By suitably controlling driving of the actuators provided in the first joint portion 5311a to sixth joint portion 5311f by the control apparatus 5317, the posture of the arm unit 5309, namely, the position and the posture of the microscope unit 5303, can be controlled. Specifically, the control apparatus 5317 can comprehend the posture of the arm unit 5309 at present and the position and the posture of the microscope unit 5303 at present on the basis of information regarding the angle of rotation of the joint portions detected by the encoders. The control apparatus 5317 uses the comprehended information to calculate a control value (for example, an angle of rotation or torque to be generated) for each joint portion with which a movement of the microscope unit 5303 in accordance with an operation input from the user is implemented. Accordingly, the control apparatus 5317 drives the driving mechanism of each joint portion in accordance with the control value. It is to be noted that, in this case, the control method of the arm unit 5309 by the control apparatus 5317 is not limited, and various known control methods such as force control or position control may be applied.

For example, when the surgeon performs operation inputting suitably through an inputting apparatus not illustrated, driving of the arm unit 5309 may be controlled suitably in response to the operation input by the control apparatus 5317 to control the position and the posture of the microscope unit 5303. By this control, it is possible to support, after the microscope unit 5303 is moved from an arbitrary position to a different arbitrary position, the microscope unit 5303 fixedly at the position after the movement. It is to be noted that, as the inputting apparatus, preferably an inputting apparatus is applied which can be operated by the surgeon even if the surgeon has a surgical tool in its hand such as, for example, a foot switch taking the convenience to the surgeon into consideration. Further, operation inputting may be performed in a contactless fashion on the basis of gesture detection or line-of-sight detection in which a wearable device or a camera which is provided in the operating room is used. This makes it possible even for a user who belongs to a clean area to operate an apparatus belonging to an unclean area with a high degree of freedom. In addition, the arm unit 5309 may be operated in a master-slave fashion. In this case, the arm unit 5309 may be remotely controlled by the user through an inputting apparatus which is placed at a place remote from the operating room.

Further, where force control is applied, the control apparatus 5317 may perform power-assisted control to drive the actuators of the first joint portion 5311a to sixth joint portion 5311f such that the arm unit 5309 may receive external force by the user and move smoothly following the external force. This makes it possible to move, when the user holds and directly moves the position of the microscope unit 5303, the microscope unit 5303 with comparatively weak force. Accordingly, it becomes possible for the user to move the microscope unit 5303 more intuitively by a simpler and easier operation, and the convenience to the user can be improved.

Further, driving of the arm unit 5309 may be controlled such that the arm unit 5309 performs a pivot movement. The pivot movement here is a motion for moving the microscope unit 5303 such that the direction of the optical axis of the microscope unit 5303 is kept toward a predetermined point (hereinafter referred to as pivot point) in a space. Since the pivot movement makes it possible to observe the same observation position from various directions, more detailed observation of an affected area becomes possible. It is to be noted that, where the microscope unit 5303 is configured such that the focal distance thereof is fixed, preferably the pivot movement is performed in a state in which the distance between the microscope unit 5303 and the pivot point is fixed. In this case, it is sufficient if the distance between the microscope unit 5303 and the pivot point is adjusted to a fixed focal distance of the microscope unit 5303 in advance. By the configuration just described, the microscope unit 5303 comes to move on a hemispherical plane (schematically illustrated in FIG. 15) having a radius corresponding to the focal distance centered at the pivot point, and even if the observation direction is changed, a clear captured image can be obtained. On the other hand, where the microscope unit 5303 is configured such that the focal distance thereof is adjustable, the pivot movement may be performed in a state in which the distance between the microscope unit 5303 and the pivot point is variable. In this case, for example, the control apparatus 5317 may calculate the distance between the microscope unit 5303 and the pivot point on the basis of information regarding the angles of rotation of the joint portions detected by the encoders and automatically adjust the focal distance of the microscope unit 5303 on the basis of a result of the calculation. Alternatively, where the microscope unit 5303 includes an AF function, adjustment of the focal distance may be performed automatically by the AF function every time the changing in distance caused by the pivot movement between the microscope unit 5303 and the pivot point.

Further, each of the first joint portion 5311a to sixth joint portion 5311f may be provided with a brake for constraining the rotation of the first joint portion 5311a to sixth joint portion 5311f. Operation of the brake may be controlled by the control apparatus 5317. For example, if it is intended to fix the position and the posture of the microscope unit 5303, then the control apparatus 5317 renders the brakes of the joint portions operative. Consequently, even if the actuators are not driven, the posture of the arm unit 5309, namely, the position and posture of the microscope unit 5303, can be fixed, and therefore, the power consumption can be reduced. When it is intended to move the position and the posture of the microscope unit 5303, it is sufficient if the control apparatus 5317 releases the brakes of the joint portions and drives the actuators in accordance with a predetermined control method.

Such operation of the brakes may be performed in response to an operation input by the user through the operation unit 5307 described hereinabove. When the user intends to move the position and the posture of the microscope unit 5303, the user would operate the operation unit 5307 to release the brakes of the joint portions. Consequently, the operation mode of the arm unit 5309 changes to a mode in which rotation of the joint portions can be performed freely (all-free mode). On the other hand, if the user intends to fix the position and the posture of the microscope unit 5303, then the user would operate the operation unit 5307 to render the brakes of the joint portions operative. Consequently, the operation mode of the arm unit 5309 changes to a mode in which rotation of the joint portions is constrained (fixed mode).

The control apparatus 5317 integrally controls operation of the microscopic surgery system 5300 by controlling operation of the microscope apparatus 5301 and the display apparatus 5319. For example, the control apparatus 5317 renders the actuators of the first joint portion 5311a to sixth joint portion 5311f operative in accordance with a predetermined control method to control driving of the arm unit 5309. Further, for example, the control apparatus 5317 controls operation of the brakes of the first joint portion 5311a to sixth joint portion 5311f to change the operation mode of the arm unit 5309. Further, for example, the control apparatus 5317 performs various signal processes for an image signal acquired by the image pickup unit of the microscope unit 5303 of the microscope apparatus 5301 to generate image data for display and controls the display apparatus 5319 to display the generated image data. As the signal processes, various known signal processes such as, for example, a development process (demosaic process), an image quality improving process (such as a bandwidth enhancement process, a super-resolution process, a noise reduction (NR) process and/or an image stabilization process) and/or an enlargement process (namely, an electronic zooming process) may be performed.

It is to be noted that communication between the control apparatus 5317 and the microscope unit 5303 and communication between the control apparatus 5317 and the first joint portion 5311a to sixth joint portion 5311f may be wired communication or wireless communication. Where wired communication is applied, communication by an electric signal may be performed or optical communication may be performed. In this case, a cable for transmission used for wired communication may be configured as an electric signal cable, an optical fiber or a composite cable of them in response to an applied communication method. On the other hand, where wireless communication is applied, since there is no necessity to lay a transmission cable in the operating room, such a situation that movement of medical staff in the operating room is disturbed by a transmission cable can be eliminated.

The control apparatus 5317 may be a processor such as a central processing unit (CPU) or a graphics processing unit (GPU), or a microcomputer or a control board in which a processor and a storage element such as a memory are incorporated. The various functions described hereinabove can be implemented by the processor of the control apparatus 5317 operating in accordance with a predetermined program. It is to be noted that, in the example illustrated, the control apparatus 5317 is provided as an apparatus separate from the microscope apparatus 5301. However, the control apparatus 5317 may be installed in the inside of the base unit 5315 of the microscope apparatus 5301 and configured integrally with the microscope apparatus 5301. The control apparatus 5317 may also include a plurality of apparatus. For example, microcomputers, control boards or the like may be disposed in the microscope unit 5303 and the first joint portion 5311a to sixth joint portion 5311f of the arm unit 5309 and connected for communication with each other to implement functions similar to those of the control apparatus 5317.

The display apparatus 5319 is provided in the operating room and displays an image corresponding to image data generated by the control apparatus 5317 under the control of the control apparatus 5317. In other words, an image of a surgical region picked up by the microscope unit 5303 is displayed on the display apparatus 5319. The display apparatus 5319 may display, in place of or in addition to an image of a surgical region, various kinds of information relating to the surgery such as physical information of a patient or information regarding a surgical procedure of the surgery. In this case, the display of the display apparatus 5319 may be switched suitably in response to an operation by the user. Alternatively, a plurality of such display apparatus 5319 may also be provided such that an image of a surgical region or various kinds of information relating to the surgery may individually be displayed on the plurality of display apparatus 5319. It is to be noted that, as the display apparatus 5319, various known display apparatus such as a liquid crystal display apparatus or an electro luminescence (EL) display apparatus may be applied.

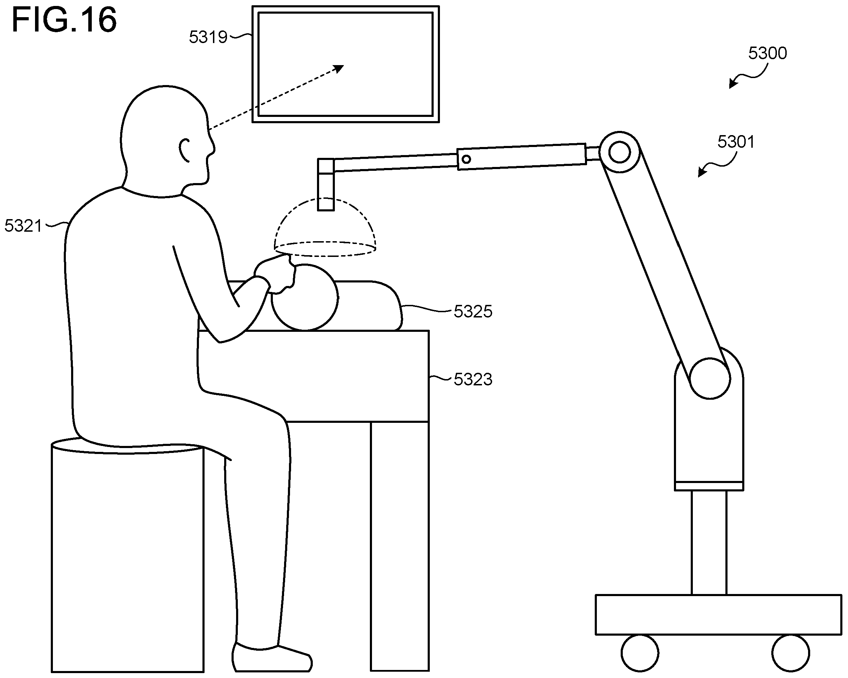

FIG. 16 is a view illustrating a state of surgery in which the microscopic surgery system 5300 illustrated in FIG. 15 is used. FIG. 16 schematically illustrates a state in which a surgeon 5321 uses the microscopic surgery system 5300 to perform surgery for a patient 5325 on a patient bed 5323. It is to be noted that, in FIG. 16, for simplified illustration, the control apparatus 5317 from among the components of the microscopic surgery system 5300 is omitted and the microscope apparatus 5301 is illustrated in a simplified form.

As illustrated in FIG. 2C, upon surgery, using the microscopic surgery system 5300, an image of a surgical region picked up by the microscope apparatus 5301 is displayed in an enlarged scale on the display apparatus 5319 installed on a wall face of the operating room. The display apparatus 5319 is installed at a position opposing to the surgeon 5321, and the surgeon 5321 would perform various treatments for the surgical region such as, for example, resection of the affected area while observing a state of the surgical region from a video displayed on the display apparatus 5319.

An example of the microscopic surgery system 5300 to which the technology according to an embodiment of the present disclosure can be applied has been described. It is to be noted here that, while the microscopic surgery system 5300 is described as an example, the system to which the technology according to an embodiment of the present disclosure can be applied is not limited to this example. For example, the microscope apparatus 5301 may also function as a supporting arm apparatus which supports, at a distal end thereof, a different observation apparatus or some other surgical tool in place of the microscope unit 5303. As the other observation apparatus, for example, an endoscope may be applied. Further, as the different surgical tool, forceps, tweezers, a pneumoperitoneum tube for pneumoperitoneum or an energy device for performing incision of a tissue or sealing of a blood vessel by cautery and so forth can be applied. By supporting any of such an observation apparatus and surgical tools as just described by the supporting apparatus, the position of them can be fixed with a high degree of stability in comparison with that in an alternative case in which they are supported by hands of medical staff. Accordingly, the burden on the medical staff can be reduced. The technology according to an embodiment of the present disclosure may be applied to a supporting arm apparatus which supports such a component as described above other than the microscopic unit.

The technology according to the present disclosure is suitably applicable to the control apparatus 5317 among the configurations described above. Specifically, the technology according to the present disclosure is applicable in the case where the image of the surgical site in the patient 5325 taken by the image pickup unit of the microscope unit 5303 is displayed on the display apparatus 5319. The technology according to the present disclosure applied to the control apparatus 5317 makes it possible, even if the motion projected in the special-light image and the motion projected in the white-light image are different, to use the respective motion vectors appropriately depending on the degree of correlation of the respective motion vectors of both images. Thus, it is possible to perform correction (such as NR processing) on the special-light image with high accuracy. This allows the surgeon 5321 to observe the surgical site image being corrected (such as being subjected to NR processed) with high accuracy in real-time on the display apparatus 5319, leading to safer surgery.

Note that the present technology may include the following configuration.

(1) A medical system comprising:

irradiation means for irradiating an image capturing target with an electromagnetic wave;
image capturing means for capturing a reflected wave caused by the image capturing target irradiated with the electromagnetic wave;
acquisition means for acquiring, from the image capturing means, a first image based on a first wavelength band and a second image based on a second wavelength band different from the first wavelength band;
first motion estimation means for calculating a first motion vector as a motion vector between a plurality of the first images on a basis of a feature value in the first image;
second motion estimation means for calculating a second motion vector as a motion vector between a plurality of the second images on a basis of a feature value in the second image;
correlation degree calculation means for calculating a degree of correlation between the first motion vector and the second motion vector; and
correction means for correcting the first image on a basis of the degree of correlation.

(2) The medical system according to (1), wherein the medical system is a microscopic surgery system or an endoscopic surgery system.
(3) An information processing apparatus comprising:

acquisition means for acquiring, from image capturing means, a first image based on a first wavelength band and a second image based on a second wavelength band different from the first wavelength band, the image capturing means capturing a reflected wave caused by an image capturing target irradiated with an electromagnetic wave;
first motion estimation means for calculating a first motion vector as a motion vector between a plurality of the first images on a basis of a feature value in the first image;
second motion estimation means for calculating a second motion vector as a motion vector between a plurality of the second images on a basis of a feature value in the second image;
correlation degree calculation means for calculating a degree of correlation between the first motion vector and the second motion vector; and
correction means for correcting the first image on a basis of the degree of correlation.

(4) The information processing apparatus according to (3), wherein
the correction means
corrects the first image on a basis of the second motion vector in a case where the degree of correlation is equal to or higher than a predetermined threshold, and
corrects the first image on a basis of the first motion vector in a case where the degree of correlation is less than the predetermined threshold.

(5) The information processing apparatus according to (3), wherein
the correction means
calculates a third motion vector by weighting and summing the first motion vector and the second motion vector depending on the degree of correlation to correct the first image on a basis of the third motion vector.

(6) The information processing apparatus according to (3), wherein
the correction means
compensates motion of the first image on a basis of the first motion vector,
compensates motion of the second image on a basis of the second motion vector, and
generates a third image by weighting and summing the first image being motion-compensated and the second image being motion-compensated depending on the degree of correlation to correct the first image on a basis of the third image.

(7) The information processing apparatus according to any of (3) to (6), wherein
the correlation degree calculation means
calculates a correlation coefficient between the first motion vector and the second motion vector as the degree of correlation.

(8) The information processing apparatus according to any of (3) to (6), wherein
the correlation degree calculation means
calculates a sum of absolute values of differences between the first motion vector and the second motion vector as the degree of correlation.

(9) The information processing apparatus according to any of (3) to (6), wherein
the correlation degree calculation means
calculates a sum of squares of differences between the first motion vector and the second motion vector as the degree of correlation.

(10) The information processing apparatus according to any of (3) to (9), wherein the correction means performs noise reduction processing for reducing noise in the first image as processing for correcting the first image on a basis of the degree of correlation.

(11) The information processing apparatus according to any of (3) to (9), wherein the correction means performs image enhancement processing for enhancing the first image as processing for correcting the first image on a basis of the degree of correlation.

(12) The information processing apparatus according to any of (3) to (11), wherein the first image is a near-infrared light image and the second image is a white-light image.

(13) An information processing method comprising:

an acquisition process of acquiring, from image capturing means, a first image based on a first wavelength band and a second image based on a second wavelength band different from the first wavelength band, the image capturing means capturing a reflected wave caused by an image capturing target irradiated with an electromagnetic wave;
a first motion estimation process of calculating a first motion vector as a motion vector between a plurality of the first images on a basis of a feature value in the first image;
a second motion estimation process of calculating a second motion vector as a motion vector between a plurality of the second images on a basis of a feature value in the second image;
a correlation degree calculation process of calculating a degree of correlation between the first motion vector and the second motion vector; and
a correction process of correcting the first image on a basis of the degree of correlation.

Although the description above is give of the embodiments and modifications of the present disclosure, the technical scope of the present disclosure is not limited to the above-described embodiments and modifications as they are, and various modifications and variations can be made without departing from the spirit and scope of the present disclosure. In addition, components covering different embodiments and modifications can be combined as appropriate.

In one example, the description of the first embodiment mainly gives NR processing as processing for correction of the special-light image performed by the correction unit 1315, but the processing for correction is not limited thereto. Other processing, such as image enhancement processing (e.g., edge enhancement processing), can be used.

Further, the degree of correlation is the only factor for determining how to use two motion vectors (e.g., the special-light motion vector and the white-light motion vector), but it is not limited to the example described above, and other factors can also be used together. In one example, the brighter the usage environment of the medical system 1, the more the use cases or rates of the white-light motion vector. In addition, the noise amount of the special-light image and the noise amount of the white-light image, which can be seen from the signal amplification factor of the IR imager or the RGB imager, can be considered.

Further, in performing NR processing by the correction unit 1315, NR processing in the spatial direction can be used in combination with NR processing in the temporal direction.

Further, the image to be used is not limited to two images (such as special-light image and white-light image), and three or more images can be used.

Moreover, the effects in each of the embodiments and modifications described in the present specification are merely illustrative and are not restrictive, and other effects are achievable.

Reference Signs List

| | |
|---|---|
| 1 | MEDICAL SYSTEM |
| 2 | IMAGE CAPTURING TARGET |
| 11 | LIGHT SOURCE |
| 12 | IMAGE CAPTURING APPARATUS |
| 13 | INFORMATION PROCESSING APPARATUS |
| 14 | DISPLAY APPARATUS |
| 131 | PROCESSING UNIT |
| 132 | STORAGE UNIT |
| 1311 | ACQUISITION UNIT |
| 1312 | FIRST MOTION ESTIMATION UNIT |
| 1313 | SECOND MOTION ESTIMATION UNIT |
| 1314 | CORRELATION DEGREE CALCULATION UNIT |
| 1315 | CORRECTION UNIT |
| 1316 | DISPLAY CONTROL UNIT |

**Claims**

1. A medical system comprising:

   irradiation means for irradiating an image capturing target with an electromagnetic wave;
   image capturing means for capturing a reflected wave caused by the image capturing target irradiated with the electromagnetic wave;
   acquisition means for acquiring, from the image capturing means, a first image based on a first wavelength band and a second image based on a second wavelength band different from the first wavelength band;
   first motion estimation means for calculating a first motion vector as a motion vector between a plurality of the first images on a basis of a feature value in the first image;
   second motion estimation means for calculating a second motion vector as a motion vector between a plurality of the second images on a basis of a feature value in the second image;
   correlation degree calculation means for calculating a degree of correlation between the first motion vector and the second motion vector; and
   correction means for correcting the first image on a basis of the degree of correlation.

2. The medical system according to claim 1, wherein the medical system is a microscopic surgery system or an endoscopic surgery system.

3. An information processing apparatus comprising:

   acquisition means for acquiring, from image capturing means, a first image based on a first wavelength band and a second image based on a second wavelength band different from the first wavelength band, the image capturing means capturing a reflected wave caused by an image capturing target irradiated with an electromagnetic wave;
   first motion estimation means for calculating a first motion vector as a motion vector between a plurality of the first images on a basis of a feature value in the first image;
   second motion estimation means for calculating a second motion vector as a motion vector between a plurality of the second images on a basis of a feature value in the second image;
   correlation degree calculation means for calculating a degree of correlation between the first motion vector and the second motion vector; and
   correction means for correcting the first image on a basis of the degree of correlation.

4. The information processing apparatus according to claim 3, wherein
   the correction means
   corrects the first image on a basis of the second motion vector in a case where the degree of correlation is equal to or higher than a predetermined threshold, and
   corrects the first image on a basis of the first motion vector in a case where the degree of correlation is less than the predetermined threshold.

5. The information processing apparatus according to claim 3, wherein
   the correction means
   calculates a third motion vector by weighting and summing the first motion vector and the second motion vector depending on the degree of correlation to correct the first image on a basis of the third motion vector.

6. The information processing apparatus according to claim 3, wherein
   the correction means
   compensates motion of the first image on a basis of the first motion vector,
   compensates motion of the second image on a basis of the second motion vector, and
   generates a third image by weighting and summing the first image being motion-compensated and the second image being motion-compensated depending on the degree of correlation to correct the first image on a basis of the third image.

7. The information processing apparatus according to claim 3, wherein
   the correlation degree calculation means
   calculates a correlation coefficient between the first motion vector and the second motion vector as the degree of correlation.

8. The information processing apparatus according to claim 3, wherein
   the correlation degree calculation means
   calculates a sum of absolute values of differences between the first motion vector and the second motion vector as the degree of correlation.

9. The information processing apparatus according to claim 3, wherein
   the correlation degree calculation means
   calculates a sum of squares of differences between the first motion vector and the second motion vector as the degree of correlation.

10. The information processing apparatus according to claim 3, wherein the correction means performs noise reduction processing for reducing noise in the first image as processing for correcting the first image on a basis of the degree of correlation.

11. The information processing apparatus according to claim 3, wherein the correction means performs image enhancement processing for enhancing the first image as processing for correcting the first image on a basis of the degree of correlation.

**12.** The information processing apparatus according to claim 3, wherein the first image is a near-infrared light image and the second image is a white-light image.

**13.** An information processing method comprising:

an acquisition process of acquiring, from image capturing means, a first image based on a first wavelength band and a second image based on a second wavelength band different from the first wavelength band, the image capturing means capturing a reflected wave caused by an image capturing target irradiated with an electromagnetic wave;

a first motion estimation process of calculating a first motion vector as a motion vector between a plurality of the first images on a basis of a feature value in the first image;

a second motion estimation process of calculating a second motion vector as a motion vector between a plurality of the second images on a basis of a feature value in the second image;

a correlation degree calculation process of calculating a degree of correlation between the first motion vector and the second motion vector; and

a correction process of correcting the first image on a basis of the degree of correlation.

# FIG.1

# FIG.2

# FIG.3

| SPECIAL LIGHT | WHITE LIGHT |
|---|---|

NOISE

SIGNAL

SIGNAL

NOISE

# FIG.4

SPECIAL WHITE
LIGHT LIGHT

MEM-
BRANE

2
IMAGE
CAPTURING
TARGET

TISSUE

MOTION

NO
MOTION

ICG
FLUORESCENCE

# FIG.5

PAST IMAGE

INPUT IMAGE

MOTION VECTOR

MOTION
COMPENSATION

MOTION
COMPENSATION
IMAGE

NR

# FIG.6

| WHITE-LIGHT IMAGE | SPECIAL-LIGHT IMAGE |
|---|---|
| | |

# FIG.7

| CASE 1 | | CASE 2 | |
|---|---|---|---|
| SPECIAL-LIGHT IMAGE | WHITE-LIGHT IMAGE | SPECIAL-LIGHT IMAGE | WHITE-LIGHT IMAGE |
| | | | |
| MOTION CORRELATION: LARGE | | MOTION CORRELATION: SMALL | |

# FIG.8

PAST IMAGE

INPUT IMAGE

MOTION COMPENSATION

MOTION COMPENSATION IMAGE

WEIGHTED AVERAGE

OUTPUT

# FIG.9

START

S1

ACQUIRE SPECIAL-LIGHT IMAGE
AND WHITE-LIGHT IMAGE

S2

CALCULATE SPECIAL-LIGHT
MOTION VECTOR IN
SPECIAL-LIGHT IMAGE

S3

CALCULATE WHITE-LIGHT
MOTION VECTOR IN
WHITE-LIGHT IMAGE

S4

CALCULATE DEGREE OF
CORRELATION BETWEEN
SPECIAL-LIGHT MOTION
VECTOR AND WHITE-LIGHT
MOTION VECTOR

S5

IS DEGREE OF
CORRELATION EQUAL TO
OR HIGHER THAN
THRESHOLD?

NO

YES  S6

PERFORM CORRECTION ON
SPECIAL-LIGHT IMAGE ON BASIS
OF WHITE-LIGHT MOTION
VECTOR

S7

PERFORM CORRECTION ON
SPECIAL-LIGHT IMAGE ON BASIS
OF SPECIAL-LIGHT MOTION
VECTOR

S8

DISPLAY SPECIAL-LIGHT IMAGE

END

# FIG.10

# FIG.11

```
                    START

                                              ┌S1
        ACQUIRE SPECIAL-LIGHT IMAGE
          AND WHITE-LIGHT IMAGE

                                              ┌S2
        CALCULATE SPECIAL-LIGHT MOTION VECTOR
             IN SPECIAL-LIGHT IMAGE

                                              ┌S3
        CALCULATE WHITE-LIGHT MOTION VECTOR
             IN WHITE-LIGHT IMAGE

                                              ┌S4
      CALCULATE DEGREE OF CORRELATION BETWEEN
      SPECIAL-LIGHT MOTION VECTOR AND WHITE-LIGHT
                   MOTION VECTOR

                                              ┌S11
      CALCULATE THIRD MOTION VECTOR BY WEIGHTING
       AND SUMMING SPECIAL-LIGHT MOTION VECTOR
      AND WHITE-LIGHT MOTION VECTOR DEPENDING ON
                DEGREE OF CORRELATION

                                              ┌S12
      PERFORM CORRECTION ON SPECIAL-LIGHT IMAGE ON
            BASIS OF THIRD MOTION VECTOR

                                              ┌S8
           DISPLAY SPECIAL-LIGHT IMAGE

                    END
```

# FIG.12

START

↓ S1

ACQUIRE SPECIAL-LIGHT IMAGE
AND WHITE-LIGHT IMAGE

↓ S2

CALCULATE SPECIAL-LIGHT MOTION VECTOR
IN SPECIAL-LIGHT IMAGE

↓ S3

CALCULATE WHITE-LIGHT MOTION VECTOR
IN WHITE-LIGHT IMAGE

↓ S4

CALCULATE DEGREE OF CORRELATION BETWEEN
SPECIAL-LIGHT MOTION VECTOR AND WHITE-LIGHT
MOTION VECTOR

↓ S21

COMPENSATE FOR MOTION OF SPECIAL-LIGHT IMAGE
ON BASIS OF SPECIAL-LIGHT MOTION VECTOR

↓ S22

COMPENSATE FOR MOTION OF WHITE-LIGHT IMAGE ON
BASIS OF WHITE-LIGHT MOTION VECTOR

↓ S23

GENERATE THIRD IMAGE BY WEIGHTING AND SUMMING
MOTION-COMPENSATED SPECIAL-LIGHT IMAGE AND
MOTION-COMPENSATED WHITE-LIGHT IMAGE
DEPENDING ON DEGREE OF CORRELATION

↓ S24

PERFORM CORRECTION ON SPECIAL-LIGHT IMAGE ON
BASIS OF THIRD IMAGE

↓ S8

DISPLAY SPECIAL-LIGHT IMAGE

↓

END

# FIG.13

# FIG.14

**CAMERA HEAD** 5005

**LENS UNIT** 5007 → **IMAGE PICKUP UNIT** 5009 → **COMMUNICA-TION UNIT** 5013

**DRIVING UNIT** 5011 ← **CAMERA HEAD CONTROLLING UNIT** 5015

5065

**CCU** 5039

**IMAGE PROCESSING UNIT** 5061

**COMMUNICA-TION UNIT** 5059

**CONTROL UNIT** 5063

FIG.15

# FIG.16

EP 3 848 895 A1

<table>
<tr><td colspan="3"><strong>INTERNATIONAL SEARCH REPORT</strong></td><td colspan="2">International application No.<br>PCT/JP2019/034310</td></tr>
</table>

| A.   CLASSIFICATION OF SUBJECT MATTER |
|---|
| Int.Cl.  G06T7/246(2017.01)i |
| According to International Patent Classification (IPC) or to both national classification and IPC |

| B.   FIELDS SEARCHED |
|---|
| Minimum documentation searched (classification system followed by classification symbols) |
| Int.Cl.  G06T7/246 |

| Documentation searched other than minimum documentation to the extent that such documents are included in the fields searched |
|---|
| Published examined utility model applications of Japan        1922-1996 |
| Published unexamined utility model applications of Japan      1971-2019 |
| Registered utility model specifications of Japan              1996-2019 |
| Published registered utility model applications of Japan      1994-2019 |

| Electronic data base consulted during the international search (name of data base and, where practicable, search terms used) |
|---|
| |

| C.   DOCUMENTS CONSIDERED TO BE RELEVANT |

| Category* | Citation of document, with indication, where appropriate, of the relevant passages | Relevant to claim No. |
|---|---|---|
| A | JP 5603676 B2 (OLYMPUS CORP.) 08 October 2014, claims 1-19 & US 2011/0317043 A1, claims 1-23 & CN 102316247 A | 1-13 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 November 2019 (01.11.2019) | 12 November 2019 (12.11.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**REFERENCES CITED IN THE DESCRIPTION**

**Patent documents cited in the description**

- JP 5603676 B